(19) Europäisches Patentamt
European Patent Office
Office européen des brevets

(11) Publication number: 0 324 210 B1

(12) # EUROPEAN PATENT SPECIFICATION

(45) Date of publication of the patent specification: 31.10.90

(51) Int. Cl.[5]: C07C 59/265, C07C 51/47

(21) Application number: 88300155.4

(22) Date of filing: 11.01.88

(54) **Separation of citric acid from fermentation broth.**

(43) Date of publication of application: 19.07.89 Bulletin 89/29

(45) Publication of the grant of the patent: 31.10.90 Bulletin 90/44

(84) Designated Contracting States: AT BE CH DE ES FR GB IT LI NL SE

(56) References cited:
CH-A- 523 861
DE-A- 2 931 759
GB-A- 868 926
US-A- 2 985 589
US-A- 3 706 812
US-A- 4 353 839

(73) Proprietor: UOP (a New York general partnership), 25 East Algonquin Road, Des Plaines Illinois 60017-5017(US)

(72) Inventor: Kulprathipanja, Santi, 3920 Winston Drive, Hoffman Estates Illinois 60172(US)

(74) Representative: Brock, Peter William, URQUHART-DYKES & LORD 91 Wimpole Street, London W1M 8AH(GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



ACTORUM AG

## Description

The field of art to which this invention pertains is the solid bed adsorptive separation of citric acid from fermentation broths containing citric acid, carbohydrates, amino acids, proteins and salts. More specifically, the invention relates to a process for separating citric acid from fermentation broths containing same which process employs a non-zeolite polymeric adsorbent, which selectively adsorb citric acid, and is selected from the group consisting of a neutral, crosslinked polystyrene polymer, a nonionic hydrophobic polyacrylic ester polymer, a weakly basic anionic exchange resin possessing tertiary amine or pyridine functional groups, and a strongly basic anionic exchange resin possessing quaternary amine functional groups and mixtures thereof

Citric acid is used as a food acidulant, and in pharmaceutical, industrial and detergent formulations. The increased popularity of liquid detergents formulated with citric acid has been primarily responsible for growth of worldwide production of citric acid to about 320 million Kg per year which is expected to continue in the future.

Citric acid is produced by a submerged culture fermentation process which employs molasses as feed and the microorganism, Aspergillus Niger. The fermentation product will contain carbohydrates, amino acids, proteins and salts as well as citric acid, which must be separated from the fermentation broth.

There are two technologies currently employed for the separation of citric acid. The first involves calcium salt precipitation of citric acid. The resulting calcium citrate is acidified with sulfuric acid. In the second process, citric acid is extracted from the fermentation broth with a mixture of trilauryl-amine, n-octanol and a $C_{10}$ or $C_{11}$ isoparaffin. Citric acid is reextracted from the solvent phase into water with the addition of heat. Both techniques, however, are complex, expensive and they generate a substantial amount of waste for disposal.

The patent literature has suggested a possible third method for separating citric acid from the fermentation broth, which involves membrane filtration to remove raw materials or high molecular weight impurities and then adsorption of contaminants onto a nonionic resin based on polystyrene or polyacrylic resins and collection of the citric acid in the rejected phase or raffinate and crystallization of the citric acid after concentrating the solution, or by precipitating the citric acid as the calcium salts then acidifying with $H_2SO_4$, separating the $CaSO_4$ and contacting cation- and anion-exchangers. This method, disclosed in EPA-A 151 470 is also a rather complex and lengthy method for separating the citric acid. In contrast, the present method makes it possible to separate the citric acid in a single adsorption step and to recover the citric acid from the adsorbent to obtain the purified citric acid using an easily separated desorbent.

This invention relates to a process for adsorbing citric acid from a fermentation broth onto a polymeric adsorbent selected from the group consisting of a neutral, crosslinked polystyrene polymer, a nonionic hydrophobic polyacrylic ester polymer, a weakly basic anionic exchange resin possessing tertiary amine or pyridine functional groups, and a strongly basic anionic exchange resin possessing quaternary amine functional groups and mixtures thereof and thereafter recovering the citric acid by desorption thereof with a suitable desorbent under desorption conditions. One aspect of the invention is in the discovery that highly selective separation of citric acid from salts and carbohydrates is only achieved by adjusting and maintaining the pH of the feed solution lower than the first ionization constant ($pKa_1$) of citric acid (3.13). The degree to which the pH must be lowered to maintain adequate selectivity appears to be interdependent on the concentration of citric acid in the feed mixture; the pH is inversely dependent on the concentration. As concentrations are decreased below 13% to very low concentrations, the pH may be near the $pKa_1$ of citric acid of 3.13; at 13%, the pH may range from 0.9 to 1.7; however, at 40% citric acid feed concentration, the pH must be lowered to at least 1.2 or lower. At higher concentrations, the pH must be even lower; for example, at 50% citric acid, the pH must be at or below 1.0. It is thus preferred to maintain the pH of the feed mixture inthe range of 0.5 to 2.5 with a range of 0.5 to 2.2 giving best results. Another aspect of the invention is the discovery that the temperature of adsorption can be reduced for the polymeric adsorbent used herein by the addition of acetone, or other low molecular weight ketone, to the desorbent; the higher temperatures associated with adsorbent breakdown can thus be avoided.

The invention also relates to a process for separating citric acid from a feed mixture comprising a fermentation broth containing same, which process employs a polymeric adsorbent selected from the group consisting of a neutral, crosslinked polystyrene polymer, a nonionic hydrophobic polyacrylic ester polymer, a weakly basic anionic exchange resin possessing tertiary amine or pyridine functional groups, and a strongly basic anionic exchange resin possessing quaternary amine functional groups and mixtures thereof which comprises the steps of:

(a) maintaining net fluid flow through a column of said adsorbent in a single direction, which column contains at least three zones having separate operational functions occurring therein and being serially interconnected with the terminal zones of said column connected to provide a continuous connection of said zones;

(b) maintaining an adsorption zone in said column, said zone defined by the adsorbent located between a feed input stream at an upstream boundary of said zone and a raffinate output stream at a downstream boundary of said zone;

(c) maintaining a purification zone immediately upstream from said adsorption zone, said purification zone defined by the adsorbent located between an extract output stream at an upstream boundary of said purification zone and said feed input stream at a downstream boundary of said purification zone;

(d) maintaining a desorption zone immediately upstream from said purification zone, said desorption zone defined by the adsorbent located between a desorbent input stream at an upstream boundary of said zone and said extract output stream at a downstream boundary of said zone;

(e) passing said feed mixture into said adsorption zone at adsorption conditions to effect the selective adsorption of said citric acid by said adsorbent in said adsorption zone and withdrawing a raffinate output stream comprising the nonadsorbed components of said fermentation broth from said adsorption zone;

(f) passing a desorbent material into said desorption zone at desorption conditions to effect the displacement of said citric acid from the adsorbent in said desorption zone;

(g) withdrawing an extract output stream comprising said citric acid and desorbent material from said desorption zone;

(h) passing at least a portion of said extract output stream to a separation means and therein separating at separation conditions at least a portion of said desorbent material; and,

(i) periodically advancing through said column of adsorbent in a downstream direction with respect to fluid flow in said adsorption zone the feed input stream, raffinate output stream, desorbent input stream, and extract output stream to effect the shifting of zones through said adsorbent and the production of extract output and raffinate output streams to produce a raffinate product having a reduced concentration of desorbent material. Further, a buffer zone may be maintained immediately upstream from said desorption zone, said buffer zone defined as the adsorbent located between the desorbent input stream at a downstream boundary of said buffer zone and the raffinate output stream at an upstream boundary of said buffer zone.

Other aspects of the invention encompass details of feed mixtures, adsorbents, desorbents and operating conditions which are hereinafter disclosed.

Figure 1 is a plot of concentration of various citric acid species versus the pH of citric acid dissociation which shows the shifting of the equilibrium point of the citric acid dissociation by varying the concentration of citric acid, citrate anions and the hydrogen ion.

Figure 2 is a static plot to determine the effect of pH on amount of citric acid that can be adsorbed by the adsorbent.

Figures 3A, 3B and 3C are the plots of the pulse tests in Example I using XAD-4 to separate citric acid from a feed containing 13% citric acid at pH's of 2.4, 1.7 and 0.9, respectively.

Figures 4A, 4B, 4C, 4D and 4E are plots of the pulse tests of Example II at pH's of 2.4, 1.7, 0.9, 2.8 and 1.4, respectively, run on different adsorbent samples.

Figures 5A and 5B are plots of the pulse tests of Example III at pH's of 2.8 and 1.4, respectively, and temperatures of 93° C.

Figures 6A, 6B and 6C are plots of the pulse tests of Example IV at pH's of 1.94, 1.13 and 0.5, respectively.

Figures 7A, 7B and 7C are plots of the pulse tests of Example V at pH's of 1.82, 0.5 and 0.3, respectively.

Figures 8A and 8B are plots of the pulse tests of Example VI at pH's of 1.5 and 1.0, respectively.

Figure 9 is a plot of the pulse test in Example VII showing the adsorption achieved at lower temperatures (93°C versus 45°C) through the incorporation of 10% acetone in the desorbent water.

Figure 10 is the plot of the pulse test in Example VIII using a weakly basic anionic exchange resin having a tertiary amine functionality in a cross-linked acrylic resin matrix to separate citric acid from a feed containing 40% citric acid at a pH of 1.6, desorbed with water.

Figures 11A, 11B and 11C are plots of the pulse tests of Example IX, at pH's of 7.0, 3.5 and 2.4, respectively.

Figures 12, 13A and 13B are the plots of the pulse test of Example X at a pH of 1.6 run on several different adsorbent samples of weakly basic anionic exchange resin possessing pyridine functionality in a cross-linked polystyrene resin matrix. The citric acid is desorbed with 0.05N sulfuric acid or water.

Figure 14 is a plot of the pulse test of Example XIII at a pH of 1.6.

Figure 15 is the plot of the pulse test of Example XIV at a pH of 2.2 run on a different adsorbent sample of a less strongly basic anionic exchange resin possessing quaternary ammonium functionality in a cross-linked polystyrene resin matrix, desorbed with dilute sulfuric acid.

At the outset the definitions of various terms used throughout the specification will be useful in making clear the operation, objects and advantages of the instant process.

A "feed mixture" is a mixture containing one or more extract components and one or more raffinate components to be separated by the present process. The term "feed stream" indicates a stream of a feed mixture which passes to the adsorbent used in the process.

An "extract component" is a compound or type of compound that is more selectively adsorbed by the adsorbent while a "raffinate component" is a compound or type of compound that is less selectively adsorbed. In this process, citric acid is an extract component and salts and carbohydrates are raffinate components. The term "desorbent material" shall mean generally a material capable of desorbing an extract component. The term "desorbent stream" or "desorbent input stream" indicates the stream through which desorbent material passes to the adsorbent. The term "raffinate stream" or "raffinate output stream" means a stream through which a raffinate component is removed from the adsorbent. The composition of the raffinate stream can vary from essentially 100% desorbent material to essentially 100% raffinate components. The term "extract stream" or "extract output stream" shall mean a stream through which an extract material which has been desorbed by a desorbent material is removed from the adsorbent. The composition of the extract stream, likewise, can vary from essentially 100% desorbent material to essentially 100% extract components. At least a portion of the extract stream and preferably at least a portion of the raffinate stream from the separation process are passed to separation means, typically fractionators, where at least a portion of desorbent material is separated to produce an extract product and a raffinate product. The terms "extract product" and "raffinate product" mean products produced by the process containing, respectively, an extract component and a raffinate component in higher concentrations than those found in the extract stream and the raffinate stream. Although it is possible by the process of this invention to produce a high purity, citric acid product at high recoveries, it will be appreciated that an extract component is never completely adsorbed by the adsorbent. Likewise, a raffinate component is never completely nonadsorbed by the adsorbent. Therefore, varying amounts of a raffinate component can appear in the extract stream and, likewise, varying amounts of an extract component can appear in the raffinate stream. The extract and raffinate streams then are further distinguished from each other and from the feed mixture by the ratio of the concentrations of an extract component and a raffinate component appearing in the particular stream. More specifically, the ratio of the concentration of citric acid to that of the less selectively adsorbed components will be lowest in the raffinate stream, next highest in the feed mixture, and the highest in the extract stream. Likewise, the ratio of the concentration of the less selectively adsorbed components to that of the more selectively adsorbed citric acid will be highest in the raffinate stream, next highest in the feed mixture, and the lowest in the extract stream.

The term "selective pore volume" of the adsorbent is defined as the volume of the adsorbent which selectively adsorbs an extract component from the feed mixture. The term "nonselective void volume" of the adsorbent is the volume of the adsorbent which does not selectively retain an extract component from the feed mixture. This volume includes the cavities of the adsorbent which contain no adsorptive sites and the interstitial void spaces between adsorbent particles. The selective pore volume and the nonselective void volume are generally expressed in volumetric quantities and are of importance in determining the proper flow rates of fluid required to be passed into an operational zone for efficient operations to take place for a given quantity of adsorbent. When adsorbent "passes" into an operational zone (hereinafter defined and described) employed in one embodiment of this process its nonselective void volume together with its selective pore volume carries fluid into that zone. The nonselective void volume is utilized in determining the amount of fluid which should pass into the same zone in a countercurrent direction to the adsorbent to displace the fluid present in the nonselective void volume. If the fluid flow rate passing into a zone is smaller than the nonselective void volume rate of adsorbent material passing into that zone, there is a net entrainment of liquid into the zone by the adsorbent. Since this net entrainment is a fluid present in nonselective void volume of the adsorbent, it in most instances comprises less selectively retained feed components. The selective pore volume of an adsorbent can in certain instances adsorb portions of raffinate material from the fluid surrounding the adsorbent since in certain instances there is competition between extract material and raffinate material for adsorptive sites within the selective pore volume. If a large quantity of raffinate material with respect to extract material surrounds the adsorbent, raffinate material can be competitive enough to be adsorbed by the adsorbent.

The feed material contemplated in this invention is the fermentation product obtained from the submerged culture fermentation of molasses by the microorganism, Aspergillus Niger. The fermentation product will have a composition exemplified by the following:

| | |
|---|---|
| Citric acid | 12.9% $^+$ 3% |
| Salts | 6,000 ppm |
| Carbohydrates (sugars) | 1% |
| Others (proteins and amino acids) | 5% |

The salts will be K, Na, Ca, Mg and Fe. The carbohydrates are sugars including glucose, xylose, mannose, oligosaccharides of DP2 and DP3 plus as many as 12 or more unidentified saccharides. The composition of the feedstock may vary from that given above and still be used in the invention. However, juices such as citrus fruit juices, are not acceptable or contemplated because other materials contained therein will be adsorbed at the same time rather than citric acid alone. Johnson, J. Sci. Food Agric., Vol 33 (3) pp 287-93.

It has now been discovered that the separation of citric acid can be enhanced significantly by adjusting the pH of the feed to a level below the first ionization constant of citric acid. The first ionization constant ($pKa_1$) of citric acid is 3.13, Handbook of Chemistry & Physics, 53rd Edition, 1972-3, CRC Press, and therefore, the pH of the citric acid feed should be below 3.13. When the pH for a 13% concentrated solution of citric acid is 2.4 or greater, for example, as in Figure 3A (Example I), citric acid "breaks through" (is desorbed) with the salts and carbohydrates at the beginning of the cycle, indicating that all the citric acid is not adsorbed. In contrast, less "break through" of citric acid is observed when the pH is 1.7 and no "break through" when the pH is 0.9 at the 13% level, for example as in Figures 3B and 3C, respectively.

In aqueous solution, unionized citric acid exists in equilibrium with the several citrateanions and hydrogen ions. This is shown in the following equations, where the acid dissociation constants, $pKa_1$, $pKa_2$ and $pKa_3$ of citric acid at 25°C are 3.13,4.74 and 5.40, respectively:

Equation 1

$$H_3CA \underset{}{\overset{pKa_1 = 3.13}{\rightleftharpoons}} H_2CA^{-1} + H^+ \underset{}{\overset{pKa_2 = 4.74}{\rightleftharpoons}} HCA^{-2} + H^+ \underset{}{\overset{pKa_3 = 5.40}{\rightleftharpoons}} CA^{-3} + H^+$$

The equilibrium point of citric acid dissociation can be shifted by varying the concentrations of citric acid, the citrate anion or the hydrogen ion. This is demonstrated in Figure 1, for the concentration of the several citric acid species in solution versus pH at 90°C. The result shows a higher percent of nonionized citric acid ($H_3CA$) at a higher hydrogen ion concentration (lower pH). Decreasing the pH (raising the $H^+$ ion concentration) will introduce more nonionized citric acid while reducing the citrate anionic species ($H_2CA^{-1}$, $HCA^{-2}$ and $CA^{-3}$) in the solution.

Based on the citric acid equilibrium and the resin properties mentioned above, nonionized citric acid will be separated from other ionic species (including citrate anions) in the fermentation broths using the resin adsorbents described. However, for a higher citric acid recovery, a lower pH solution is required. The static adsorption isotherm of a particular resin falling within the invention, Amberlite XAD-4, for citric acid was carried out at room temperature about 25°C, as a function of feed pH. Figure 2 shows the results of the study. The results show adsorption of the nonionic citric acid as the pH is lowered. Without the intention of being limited by this explanation, it appears that the nonionic citric acid species in the solution is preferentially adsorbed on the adsorbents of the present invention either through an acid-base interaction mechanism or a hydrogen bonding mechanism or a mechanism based on a strong affinity for relatively hydrophobic species or a combination of these mechanisms.

Desorbent materials used in various prior art adsorptive separation processes vary depending upon such factors as the type of operation employed. In the swing bed system, in which the selectively adsorbed feed component is removed from the adsorbent by a purge stream, desorbent selection is not as critical and desorbent materials comprising gaseous hydrocarbons such as methane, ethane, etc., or other types of gases such as nitrogen or hydrogen may be used at elevated temperatures or reduced pressures or both to effectively purge the adsorbed feed component from the adsorbent. However, in adsorptive separation processes which are generally operated continuously at substantially constant pressures and temperatures to ensure liquid phase, the desorbent material must be judiciously selected to satisfy many criteria. First, the desorbent material should displace an extract component from the adsorbent with reasonable mass flow rates without itself being so strongly adsorbed as to unduly prevent an extract component from displacing the desorbent material in a following adsorption cycle. Expressed in terms of the selectivity (hereinafter discussed in more detail), it is preferred that the adsorbent be more selective for all of the extract components with respect to a raffinate component than it is for the desorbent material with respect to a raffinate component. Secondly, desorbent materials must be compatible with the particular adsorbent and the particular feed mixture. More specifically, they must not reduce or destroy the critical selectivity of the adsorbent for an extract component with respect to a raffinate component. Desorbent materials should additionally be substances which are easily separable from the feed mixture that is passed into the process. Both the raffinate stream and the extract stream are removed from the adsorbent in admixture with desorbent material and without a method of separating at

least a portion of the desorbent material the purity of the extract product and the raffinate product would not be very high, nor would the desorbent material be available for reuse in the process. It is therefore contemplated that any desorbent material used in this process will preferably have a substantially different average boiling point than that of the feed mixture to allow separation of at least a portion of the desorbent material from feed components in the extract and raffinate streams by simple fractional distillation thereby permitting reuse of desorbent material in the process. The term "substantially different" as used herein shall mean that the difference between the average boiling points between the desorbent material and the feed mixture shall be at least about 5°C. The boiling range of the desorbent material may be higher or lower than that of the feed mixture. Finally, desorbent materials should also be materials which are readily available and therefore reasonable in cost. In the preferred isothermal, isobaric, liquid phase operation of the process of the present invention, it has been found that water is a particularly effective desorbent material. Also, it has been determined that acetone and other low molecular weight ketones, such as methylethyl ketone and diethyl ketone to be effective in admixture with water in small amounts, up to 15 wt.%. The key to their usefulness lies in their solubility in water. Their advantage, however, lies in their ability to reduce the temperature at which the desorption can take place With some adsorbates and water as desorbent, the temperature must be raised to aid the desorption step. Increased temperatures can cause premature deactivation of the adsorbent. A solution to that problem in this particular separation is to add acetone in the amount of 1 to 15 wt.% of the desorbent, preferably, 1 to l0 wt.% with the most preferred range of 5 to 10 wt.%. The low molecular weight ketone may also affect the adsorbent stability in possibly two ways, by removing solubilizing components which cause deactivation or by effecting regeneration, i.e., by removing the deactivating agent or reversing its effect. For example, a reduction of the desorption temperature of this separation by approximately 50°C has been achieved by adding 10 wt.% acetone to the desorbent. A reduction of from about 5°C to about 70°C can be achieved by the addition of 1 to 15 wt.% acetone to the water desorbent. Dilute inorganic acids have also been found to give good results when used as desorbents. Aqueous solutions of sulfuric acid, nitric acid, hydrochloric acid, phosphoric acid and mixtures thereof can be used in amounts corresponding to 0.01 to 1.0N (normal), with best results obtained with dilute sulfuric acid at 0.01 to 1.0N.

The prior art has also recognized that certain characteristics of adsorbents are highly desirable, if not absolutely necessary, to the successful operation of a selective adsorption process. Such characteristics are equally important to this process. Among such characteristics are: (1) adsorptive capacity for some volume of an extract component per volume of adsorbent; (2) the selective adsorption of an extract component with respect to a raffinate component and the desorbent material; and (3) sufficiently fast rates of adsorption and desorption of an extract component to and from the adsorbent. Capacity of the adsorbent for adsorbing a specific volume of an extract component is, of course, a necessity; without such capacity the adsorbent is useless for adsorptive separation. Furthermore, the higher the adsorbent's capacity for an extract component the better is the adsorbent. Increased capacity of a particular adsorbent makes it possible to reduce the amount of adsorbent needed to separate an extract component of known concentration contained in a particular charge rate of feed mixture. A reduction in the amount of adsorbent required for a specific adsorptive separation reduces the cost of the separation process. It is important that the good initial capacity of the adsorbent be maintained during actual use in the separation process over some economically desirable life. The second necessary adsorbent characteristic is the ability of the adsorbent to separate components of the feed; or, in other words, that the adsorbent possess adsorptive selectivity, (B), for one component as compared to another component. Relative selectivity can be expressed not only for one feed component as compared to another but can also be expressed between any feed mixture component and the desorbent material. The selectivity, (B), as used throughout this specification is defined as the ratio of the two components of the adsorbed phase over the ratio of the same two components in the unadsorbed phase at equilibrium conditions. Relative selectivity is shown as Equation 2 below:

Equation 2

$$\text{Selectivity} = (B) = \frac{[\text{vol. percent C/vol. percent D}]_A}{[\text{vol. percent C/vol. percent D}]_U}$$

where C and D are two components of the feed represented in volume percent and the subscripts A and U represent the adsorbed and unadsorbed phases respectively. The equilibrium conditions were determined when the feed passing over a bed of adsorbent did not change composition after contacting the bed of adsorbent. In other words, there was no net transfer of material occurring between the unadsorbed and adsorbed phases. Where selectivity of two components approaches 1.0 there is no preferential adsorption of one component by the adsorbent with respect to the other; they are both adsorbed (or nonadsorbed) to about the same degree with respect to each other. As the (B) becomes less than or greater than 1.0 there is a preferential adsorption by the adsorbent for one component with respect to the other. When comparing the selectivity by the adsorbent of one component C over component D, a (B) larger than 1.0 indicates preferential adsorption of component C within the adsorbent. A (B) less than

1.0 would indicate that component D is preferentially adsorbed leaving an unadsorbed phase richer in component C and an adsorbed phase richer in component D. Ideally desorbent materials should have a selectivity equal to about 1 or slightly less than 1 with respect to all extract components so that all of the extract components can be desorbed as a class with reasonable flow rates of desorbent material and so that extract components can displace desorbent material in a subsequent adsorption step. While separation of an extract component from a raffinate component is theoretically possible when the selectivity of the adsorbent for the extract component with respect to the raffinate component is greater than 1, it is preferred that such selectivity approach a value of 2. Like relative volatility, the higher the selectivity, the easier the separation is to perform. Higher selectivities permit a smaller amount of adsorbent to be used. The third important characteristic is the rate of exchange of the extract component of the feed mixture material or, in other words, the relative rate of desorption of the extract component. This characteristic relates directly to the amount of desorbent material that must be employed in the process to recover the extract component from the adsorbent; faster rates of exchange reduce the amount of desorbent material needed to remove the extract component and therefore permit a reduction in the operating cost of the process. With faster rates of exchange, less desorbent material has to be pumped through the process and separated from the extract stream for reuse in the process.

Resolution is a measure of the degree of separation of a two-component system, and can assist in quantifying the effectiveness of a particular combination of adsorbent, desorbent, conditions, etc. for a particular separation. Resolution for purposes of this application is defined as the distance between the two peak centers divided by the average width of the peaks at 1/2 the peak height as determined by the pulse tests described hereinafter. The equation for calculating resolution is thus:

Equation 3

$$R = \frac{L_2 - L_1}{1/2\,(W_1 + W_2)}$$

where $L_1$ and $L_2$ are the distance, in ml, respectively, from a reference point, e.g., zero to the centers of the peaks and $W_1$ and $W_2$ are the widths of the peaks at 1/2 the height of the peaks.

A dynamic testing apparatus is employed to test various adsorbents with a particular feed mixture and desorbent material to measure the adsorbent characteristics of adsorptive capacity, selectivity and exchange rate. The apparatus consists of an adsorbent chamber comprising a helical column of approximately 70 cc volume having inlet and outlet portions at opposite ends of the chamber. The chamber is contained within a temperature control means and, in addition, pressure control equipment is used to operate the chamber at a constant predetermined pressure. Quantitative and qualitative analytical equipment such as refractometers, polarimeters and chromatographs can be attached to the outlet line of the chamber and used to detect quantitatively or determine qualitatively one or more components in the effluent stream leaving the adsorbent chamber. A pulse test, performed using this apparatus and the following general procedure, is used to determine selectivities and other data for various adsorbent systems. The adsorbent is filled to equilibrium with a particular desorbent material by passing the desorbent material through the adsorbent chamber. At a convenient time, a pulse of feed containing known concentrations of a tracer and of a particular extract component or of a raffinate component or both, all diluted in desorbent, is injected for a duration of several minutes. Desorbent flow is resumed, and the tracer and the extract component or the raffinate component (or both) are eluted as in a liquid-solid chromatographic operation. The effluent can be analyzed onstream or, alternatively, effluent samples can be collected periodically and later analyzed separately by analytical equipment and traces of the envelopes of corresponding component peaks developed.

From information derived from the test adsorbent, performance can be in terms of void volume, retention volume for an extract or a raffinate component, selectivity for one component with respect to the other, and the rate of desorption of an extract component by the desorbent. The retention volume of an extract or a raffinate component may be characterized by the distance between the center of the peak envelope of an extract or a raffinate component and the peak envelope of the tracer component or some other known reference point. It is expressed in terms of the volume in cubic centimeters of desorbent pumped during this time interval represented by the distance between the peak envelopes. Selectivity, (B), for an extract component with respect to a raffinate component may be characterized by the ratio of the distance between the center of the extract component peak envelope and the tracer peak envelope (or other reference point) to the corresponding distance between the center of the raffinate component peak envelope and the tracer peak envelope. The rate of exchange of an extract component with the desorbent can generally be characterized by the width of the peak envelopes at half intensity. The narrower the peak width, the faster the desorption rate. The desorption rate can also be characterized by the distance between the center of the tracer peak envelope and the disappearance of an extract component which has just been desorbed. This distance is again the volume of desorbent pumped during this time interval.

To further evaluate promising adsorbent systems and to translate this type of data into a practical separation process requires actual testing of the best system in a continuous countercurrent liquid-solid contacting device. The general operating principles of such a device have been previously described and are found in Broughton U.S. Patent 2,985,589. A specific laboratory size apparatus utilizing these principles is described in deRosset et al., U.S. 3,706,812. The equipment comprises multiple adsorbent beds with a number of access lines attached to distributors within the beds and terminating at a rotary distributing valve. At a given valve position, feed and desorbent are being introduced through two of the lines and the raffinate and extract streams are being withdrawn through two more. All remaining access lines are inactive and when the position of the distributing valve is advanced by one index, all active positions will be advanced by one bed. This simulates a condition in which the adsorbent physically moves in a direction countercurrent to the liquid flow. Additional details on the above-mentioned nonionic adsorbent testing apparatus and adsorbent evaluation techniques may be found in the paper "Separation of $C_8$ Aromatics by Adsorption" by A. J. deRosset, R. W. Neuzil, D. J. Korous, and D. H. Rosback presented at the American Chemical Society, Los Angeles, California, March 28 through April 2, 1971.

One class of adsorbents to be used in the process of this invention will comprise nonionogenic, hydrophobic, water-insoluble, crosslinked styrene-poly(vinyl)benzene copolymers and copolymers thereof with monoethylenically unsaturated compounds or polyethylenically unsaturated monomers other than poly(vinyl)benzenes, including the acrylic esters, such as those described in Gustafson U.S. Patent Nos. 3,531,463 and 3,663,467, both incorporated herein by reference, although not limited thereto. As stated in Patent No. 3,531,463, the polymers may be made by techniques disclosed in U.S. Patent Nos. 4,221,871; 4,224,415; 4,256,840; 4,297,220; 4,382,124 and 4,501,826

Adsorbents such as just described are manufactured by the Rohm and Haas Company, and sold under the trade name "Amberlite." The types of Amberlite polymers known to be effective for use by this invention are referred to in Rohm and Haas Company literature as Amberlite adsorbents XAD-1, XAD-2, XAD-4, XAD-7 and XAD-8, and described in the literature as "hard, insoluble spheres of high surface, porous polymer." The various types of Amberlite polymeric adsorbents differ somewhat in physical properties such as porosity volume percent, skeletal density and nominal mesh sizes, but more so in surface area, average pore diameter and dipole moment. The preferred adsorbents will have a surface area of 10-2000 square meters per gram and preferably from 100-1000 $m^2/g$. These properties are listed in the following table:

TABLE 1

Properties of Amberlite Polymeric Adsorbents

| Chemical Nature | XAD-1 Poly-styrene | XAD-2 Polystyrene | XAD-4 Polystyrene | XAD-7 Acrylic Ester | XAD-8 Acrylic Ester |
|---|---|---|---|---|---|
| Porosity Volume % | 37 | 42 | 51 | 55 | 52 |
| True Wet Density grams/cc | 1.02 | 1.02 | 1.02 | 1.05 | 1.09 |
| Surface Area $M^2$/gram | 100 | 300 | 780 | 450 | 160 |
| Average Pore Diameter Angstroms | 200 | 90 | 50 | 90 | 225 |
| Skeletal Density grams/cc | 1.07 | 1.07 | 1.08 | 1.24 | 1.23 |
| Nominal Mesh Size | 20-50 | 20-50 | 20-50 | 20-50 | 25-50 |
| Dipole Moment of Functional Groups | 0.3 | 0.3 | 0.3 | 1.8 | 1.8 |

Applications for Amberlite polymeric adsorbents suggested in the Rohm and Haas Company literature include decolorizing pulp mill bleaching effluent, decolorizing dye wastes and removing pesticides from waste effluent. There is, of course, no hint in the literature of my surprising discovery of the effectiveness of Amberlite polymeric adsorbents in the separation of citric acid from Aspergillus-Niger fermentation broths.

A second class of adsorbents to be used in the process of this invention will comprise weakly basic anion exchange resins possessing tertiary amine or pyridine functionality in a cross-linked polymeric matrix, e.g., acrylic or styrene. They are especially suitable when produced in bead form, have a high degree of uniform polymeric porosity, exhibit chemical and physical stability and good resistance to attrition (not common to macroreticular resins).

Adsorbents such as just described are manufactured by the Rohm and Haas Company, and sold under the trade name "Amberlite." The types of Amberlite polymers known to be effective for use by this invention are referred to in Rohm and Haas Company literature as Amberlite adsorbents XE-275 (IRA-35), IRA-68, and described in the literature as "insoluble in all common solvents and having open structure for effective adsorption and desorption of large molecules without loss of capacity, due to organic fouling." Also, suitable are AG3-X4A and AG4-X4 manufactured by Bio Rad and comparable resins sold by Dow Chemical Co., such as Dowex 66, and Dow experimental resins made in accordance with U.S. Patents 4,031,038 and 4,098,867.

The various types of polymeric adsorbents of these classes available, will differ somewhat in physical properties such as porosity volume percent, skeletal density and nominal mesh sizes, and perhaps more so in surface area, average pore diameter and dipole moment. The preferred adsorbents will have a surface area of 10-2000 square meters per gram and preferably from 100-1000 $m^2/g$. Specific properties of the materials listed above can be found in company literature and technical brochures, such as those in the following Table 2 which are incorporated herein by reference. Others of the general class are also available.

TABLE 2

| Adsorbent | Matrix Type | Reference to Company Literature |
|---|---|---|
| AG3-4A (Bio Rad) | Polystyrene | Chromatography Electrophoresis Immunochemistry Molecular Biology - HPLC - Price List M April 1987 (Bio-Rad) |
| AG4-X4 | Acrylic | Chromatography Electrophoresis Immunochemistry Molecular Biology - HPLC - Price List M April 1987 (Bio-Rad) |
| Dow Experimental Resins | Polystyrene | U.S. Patent Nos. 4,031,038 and 4,098,867 |
| Dowex 66 | Polystyrene | Material Safety Data Sheet Printed 2/17/87 (Dow Chemical USA) |
| IRA-35 (XE-275) | Acrylic | Amberlite Ion Exchange Resins (XE-275) Rohm & Haas Co. 1975 |
| IRA-68 | Acrylic | Amberlite Ion Exchange Resins - Amberlite IRA-68 Rohm & Haas Co. April 1977 |

Applications for Amberlite polymeric adsorbents suggested in the Rohm and Haas Company literature include decolorizing pulp mill bleaching effluent, decolorizing dye wastes and removing pesticides from waste effluent. There is, of course, no hint in the literature of my surprising discovery of the effectiveness of Amberlite polymeric adsorbents in the separation of citric acid from Aspergillus-Niger fermentation broths.

A third class of adsorbents to be used in the process of this invention will comprise strongly basic anion exchange resins possessing quaternary ammonium functionality in a cross-linked polymeric matrix, e.g., divinylbenzene cross-linked acrylic or styrene resins. They are especially suitable when produced in bead form and have a high degree of uniform polymeric porosity and exhibit chemical and physical stability. In the instant case, the resins can be gelular (or "gel-type") or "macroreticular" as the term is used in some recent literature, namely Kunin and Hetherington, A Progress Report on the Removal of Colloids From Water by Macroreticular Ion Exchange Resins, paper presented at the International Water Conference, Pittsburg, PA, October 1969, reprinted by Rohm & Haas Co. In recent adsorption technology, "the term microreticular refers to the gel structure per se, size of the pores which are of atomic dimensions and depend upon the swelling properties of the gel" while "macroreticular pores and true porosity refer to structures in which the pores are larger than atomic distances and are not part of the gel structure. Their size and shape are not greatly influenced by changes in the environmental conditions such as those that result in osmotic pressure variations" while the dimensions of gel structure are "markedly dependent upon the environmental conditions." In "classical adsorption" "the terms microporous and macroporous normally refer to those pores less than 20 A and greater than 200 A, respectively. Pores of diameters between 20 A and 200 A are referred to as transitional pores." The authors selected the term "macroreticular", instead, to apply to the new ion exchange resins used in this invention, which "have both a microreticular as well as a macroreticular pore structure. The former refers to the distances between the chains and crosslinks of the swollen gel structure and the latter to the pores that are not part of the actual chemical structure. The macroreticular portion of structure may actually consist of micro-, macro-, and transitional-pores depending upon the pore size distribution." (Quotes are from page 1 of the Kunin et al. article). The macroreticular structured adsorbents also have good resistance to attrition

(not common to conventional macroreticular resins). In this application, therefore, all reference to "macroreticular" indicates adsorbent of the types described above having the dual porosity defined by Kunin and Hethesing. "Gel" and "gel-type" are used in their conventional sense.

Looking at both the quaternary ammonium function-containing ion exchange resins of the invention, the quaternary amine has a positive charge and can form an ionic bond with the sulfate ion. The sulfate form of quaternary ammonium anion exchange resin has a weakly basic property, which in turn, can adsorb citric acid through an acid-base interaction.

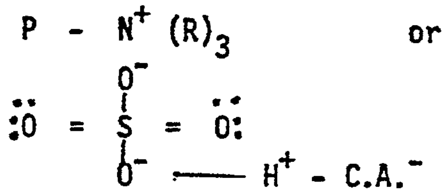

or

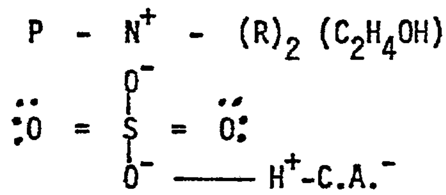

where P = resinous moiety

R = lower alkyl, $C_{1-3}$

C.A. = citrate ion

Adsorbents such as just described are manufactured by the Rohm and Haas Company, and sold under the trade name "Amberlite." The types of Amberlite polymers known to be effective for use by this invention are referred to in Rohm and Haas Company literature as Amberlite IRA 400 and 900 series adsorbents described in the literature as "insoluble in all common solvents, open structure for effective adsorption and desorption of large molecules without loss of capacity, due to organic fouling." Also suitable are AG1, AG2 and AGMP-1 resins manufactured by Bio Rad and comparable resins sold by Dow Chemical Co., such as Dowex 1, 2, 11, MSA-1 and MSA-2, etc. Also useful in this invention are the so-called intermediate base ion exchange which are mixtures of strong and weak base exchange resins. Among these are the following commercially available resins: Bio-Rex 5 (Bio-Rad 1); Amberlite IRA-47 and Duolite A-340 (both Rohm & Haas). For example, they may be useful where a basic ion exchange resin is needed which is not as basic as the strong base resins, or one which is more basic than the weakly basic resins.

The various types of polymeric adsorbents of these classes available will differ somewhat in physical properties such as porosity volume percent, skeletal density and nominal mesh sizes, and perhaps more so in surface area, average pore diameter and dipole moment. The preferred adsorbents will have a surface area of 10-2000 square meters per gram and preferably from 100-1000 $m^2/g$. Specific properties of the materials listed above can be found in company literature and technical brochures, such as those mentioned in the following Table 3

TABLE 1

PROPERTIES OF ADSORBENTS

| Adsorbent | Matrix Resin Type | Reference to Company Literature |
|---|---|---|
| IRA 458 (Rohm & Haas) | Acrylic gel-type | Amberlite Ion Exchange Resins 1986 & Technical Bulletin IE-207-74 84 |
| IRA 958 | Acrylic macroporous | Technical Bulletin and Material Safety Data Sheet are available |
| IRA 900 | Polystyrene macroporous | Technical Bulletin is available and Amberlite Ion Exchange Resins, IE-100-66. |
| IRA 904 | Polystyrene macroporous | Technical Bulletin, 1979 and IE-208/74, Jan. 1974 |
| IRA 910 | Polystyrene macroporous | Technical Bulletin, 1979 and IE-101-66, May 1972 |
| IRA 400, 402 | Polystyrene macroporus | Amberlite Ion Exchange Resins, Oct., Sept. 1976, April 1972 and IE-69-62, October 1976 |
| IRA 410 | Polystyrene gel-type | Amberlite Ion Exchange Resins IE-72-63, August 1970 |
| AG 1 (Bio Rad) | Polystyrene gel-type | Chromatography Electrophoresis Immunochemistry Molecular Biology HPLC, Price List M April 1987 |
| AG 2 | Polystyrene gel-type | Chromatography Electrophoresis Immunochemistry Molecular Biology HPLC, Price List M April 1987 |
| AG-MP-1 | Polystyrene macroporous | Chromatography Electrophoresis Immunochemistry Molecular Biology HPLC, Price List M April 1987 |
| Bio Rex 5 (Bio Rad) | Mixture of strong base and weak base resins (e.g. AG-2 and AG-3 or AG-4 | Chromatography Electrophoresis Immunochemistry Molecular Biology HPLC, Price List M April 1987 |

The adsorbent may be employed in the form of a dense compact fixed bed which is alternatively contacted with the feed mixture and desorbent materials. In the simplest embodiment of the invention the adsorbent is employed in the form of a single static bed in which case the process is only semicontinuous. In another embodiment a set of two or more static beds may be employed in fixed bed contacting with appropriate valving so that the feed mixture is passed through one or more adsorbent beds while the desorbent materials can be passed through one or more of the other beds in the set. The flow of feed mixture and desorbent materials may be either up or down through the desorbent. Any of the conventional apparatus employed in static bed fluid-solid contacting may be used.

Countercurrent moving bed or simulated moving bed countercurrent flow systems, however, have a much greater separation efficiency than fixed adsorbent bed systems and are therefore preferred. In the moving bed or simulated moving bed processes the adsorption and desorption operations are continuously taking place which allows both continuous production of an extract and a raffinate stream and the continual use of feed and desorbent streams. One preferred embodiment of this process utilizes what is known in the art as the simulated moving bed countercurrent flow system. The operating principles and sequence of such a flow system are described in U.S. Patent 2,985,589

In such a system it is the progressive movement of multiple liquid access points down an adsorbent chamber that simulates the upward movement of adsorbent contained in the chamber. Only four of the access lines are active at any one time; the feed input stream, desorbent inlet stream, raffinate outlet stream, and extract outlet stream access lines. Coincident with this simulated upward movement of the solid adsorbent is the movement of the liquid occupying the void volume of the packed bed of adsorbent. So that countercurrent contact is maintained, a liquid flow down the adsorbent chamber may be provided by a pump. As an active liquid access point moves through a cycle, that is, from the top of the chamber to the bottom, the chamber circulation pump moves through different zones which require different flow rates. A programmed flow controller may be provided to set and regulate these flow rates.

The active liquid access points effectively divided the adsorbent chamber into separate zones, each of which has a different function. In this embodiment of my process it is generally necessary that three separate operational 2 zones be present in order for the process to take place although in some instances an optional fourth zone may be used.

The adsorption zone, zone **1**, is defined as the adsorbent located between the feed inlet stream and the raffinate outlet stream. In this zone, the feedstock contacts the adsorbent, extract component is adsorbed, and a raffinate stream is withdrawn. Since the general flow through zone **1** is from the feed stream which passes into the zone to the raffinate stream which passes out of the zone, the flow in this zone is considered to be a downstream direction when proceeding from the feed inlet to the raffinate outlet streams.

Immediately upstream with respect to fluid flow in zone **1** is the purification zone, zone **2**. The purification zone is defined as the adsorbent between the extract outlet stream and the feed inlet stream. The basic operations taking place in zone **2** are the displacement from the nonselective void volume of the adsorbent of any raffinate material carried into zone **2** by shifting of adsorbent into this zone and the desorption of any raffinate material adsorbed within the selective pore volume of the adsorbent or adsorbed on the surfaces of the adsorbent particles. Purification is achieved by passing a portion of extract stream material leaving zone **3** into zone **2** at zone **2**'s upstream boundary, the extract outlet stream, to effect the displacement of raffinate material. The flow of material in zone **2** is in a downstream direction from the extract outlet stream to the feed inlet stream.

Immediately upstream of zone **2** with respect to the fluid flowing in zone **2** is the desorption zone or zone **3**. The desorption zone is defined as the adsorbent between the desorbent inlet and the extract outlet stream. The function of the desorption zone is to allow a desorbent material which passes into this zone to displace the extract component which was adsorbed upon the adsorbent during a previous contact with feed in zone **1** in a prior cycle of operation. The flow of fluid in zone **3** is essentially in the same direction as that of zones **1** and **2**.

In some instances an optional buffer zone, zone **4**, may be utilized This zone, defined as the adsorbent between the raffinate outlet stream and the desorbent inlet stream, if used, is located immediately upstream with respect to the fluid flow to zone **3**. Zone **4** would be utilized to conserve the amount of desorbent utilized in the desorption step since a portion of the raffinate stream which is removed from zone **1** can be passed into zone **4** to displace desorbent material present in that zone out of that zone into the desorption zone. Zone **4** will contain enough adsorbent so that raffinate material present in the raffinate stream passing out of zone **1** and into zone **4** can be prevented from passing into zone **3** thereby contaminating extract stream removed from zone **3**. In the instances which the fourth operational zone is not utilized the raffinate stream passed from zone **1** to zone **4** must be carefully monitored in order that the flow directly from zone **1** to zone **3** can be stopped when there is an appreciable quantity of raffinate material present in the raffinate stream passing from zone **1** into zone **3** so that the extract outlet stream is not contaminated.

A cyclic advancement of the input and output streams through the fixed bed of adsorbent can be accomplished by utilizing a manifold system in which the valves in the manifold are operated in a sequential manner to effect the shifting of the input and output streams thereby allowing a flow of fluid with respect to solid adsorbent in a countercurrent manner. Another mode of operation which can effect the countercurrent flow of solid adsorbent with respect to fluid involves the use of a rotating disc valve in which the input and output streams are connected to the valve and the lines through which feed input, extract output, desorbent input and raffinate output streams pass are advanced in the same direction through the adsorbent bed. Both the manifold arrangement and disc valve are known in the art. Specifically rotary

disc valves which can be utilized in this operation can be found in U.S. Patents 3,040,777 and 3,422,848. Both of the aforementioned patents disclose a rotary type connection valve in which the suitable advancement of the various input and output streams from fixed sources can be achieved without difficulty.

In many instances, one operational zone will contain a much larger quantity of adsorbent than some other operational zone. For instance, in some operations the buffer zone can contain a minor amount of adsorbent as compared to the adsorbent required for the adsorption and purification zones. It can also be seen that in instances in which desorbent is used which can easily desorb extract material from the adsorbent that a relatively small amount of adsorbent will be needed in a desorption zone as compared to the adsorbent needed in the buffer zone or adsorption zone or purification zone or all of them. Since it is not required that the adsorbent be located in a single column, the use of multiple chambers or a series of columns is within the scope of the invention.

It is not necessary that all of the input or output streams be simultaneously used, and in fact, in many instances one of the streams can be shut off while others effect an input or output of material. The apparatus which can be utilized to effect the process of this invention can also contain a series of individual beds connected by connecting conduits upon which are placed input or output taps to which the various input or output streams can be attached and alternately and periodically shifted to effect continuous operation. In some instances, the connecting conduits can be connected to transfer taps which during the normal operations do not function as a conduit through which material passes into or out of the process.

It is contemplated that at least a portion of the extract output stream will pass into a separation means wherein at least a portion of the desorbent material can be separated to produce an extract product containing a reduced concentration of desorbent material. Preferably, but not necessary to the operation of the process, at least a portion of the raffinate output stream will also be passed to a separation means wherein at least a portion of the desorbent material can be separated to produce a desorbent stream which can be reused in the process and a raffinate product containing a reduced concentration of desorbent material. The separation means will typically be a fractionation column, the design and operation of which is well-known to the separation art.

Reference can be made to D. B. Broughton U.S. Patent 2,985,589, and to a paper entitled "Continuous Adsorptive Processing--A New Separation Technique" by D. B. Broughton presented at the 34th Annual Meeting of the Society of Chemical Engineers at Tokyo, Japan on April 2, 1969, for further explanation of the simulated moving bed countercurrent process flow scheme.

Although both liquid and vapor phase operations can be used in many adsorptive separation processes, liquid-phase operation is preferred for this process because of the lower temperature requirements and because of the higher yields of extract product than can be obtained with liquid-phase operation over those obtained with vapor-phase operation. Adsorption conditions will include a temperature range of from 20°C to 200°C with 65°C to 100°C being more preferred and a pressure range of from atmospheric to 500 psig (3450 kPa gauge) being more preferred to ensure liquid phase. Desorption conditions will include the same range of temperatures and pressures as used for adsorption conditions.

The size of the units which can utilize the process of this invention can vary anywhere from those of pilot plant scale (see for example our assignee's U.S. Patent 3,706,812,) to those of commercial scale and can range in flow rates from as little as a few cc an hour up to many thousands of gallons per hour.

The following examples are presented to illustrate the selectivity relationship that makes the process of my invention possible. The example is not intended to unduly restrict the scope and spirit of claims attached hereto.

EXAMPLE I

In this example, three pulse tests were run with a neutral styrene divinylbenzene polymeric adsorbent (XAD-4 made by Rohm & Haas Company) to determine the ability of the adsorbent to separate citric acid, at different pH's, from its fermentation mixture of carbohydrates (DP1, DP2, DP3, including glucose, xylose, arabinose and raffinose) and ions of salts, including $Na^+$,$K^+$,$Mg^{++}$, $Ca^{++}$,$Fe^{+++}$,$Cl^-$, $SO_4^=$,$PO_4^\equiv$ and $NO_3^-$, amino acids and proteins. The first test was run at a pH of 2.4 and 45°C. Two further tests were run at a pH of 1.7 and 0.9. Citric acid was desorbed with water. The fermentation feed mixture had the following composition:

| Feed Composition | Amount |
|---|---|
| Citric Acid | 12.9% |
| Salts ($K^+$, $Na^+$, $Ca^{++}$, $Mg^{++}$ $Fe^{+++}$) | 0.60% (6000 ppm) |
| Carbohydrates (Sugars) | 1% |
| Others ($SO_4^{=}$, $Cl^-$, $PO_4^{\equiv}$, $NO_3^-$, proteins and amino acids) | 5% |
| Water | 81.5% |

Retention volumes and resolution were obtained using the pulse test apparatus and procedure previously described. Specifically, the adsorbent was tested in a 70 cc straight column using the following sequence of operations for the pulse test. Desorbent material was continuously run upwardly through the column containing the adsorbent at a nominal liquid hourly space velocity (LHSV) of about 1.0. A void volume was determined by observing the volume of desorbent required to fill the packed dry column. At a convenient time the flow of desorbent material was stopped, and a 10 cc sample of feed mixture was injected into the column via a sample loop and the flow of desorbent material was resumed. Samples of the effluent were automatically collected in an automatic sample collector and later analyzed for salts and citric acid by chromatographic analysis. Some later samples were also analyzed for carbohydrates, but since they were eluted at approximately the same rate as the carbohydrates, they were not analyzed in these examples nor were other minor ingredients, amino acids and proteins. From the analysis of these samples, peak envelope concentrations were developed for the feed mixture components. The retention volume for the citric acid was calculated by measuring the distance from the midpoint of the net retention volume of the salt envelope as the reference point to the midpoint of the citric acid envelope. The resolution, R, is calculated from Equation 3, given earlier. The separation factor, B, was calculated as previously indicated.

The results for these pulse tests are shown in the following Table 4.

TABLE 4

Test A - pH - 2.4

| Component | Net retention Volume | Peak Width at 0.5 Height | Resolution (0.5 Height) |
|---|---|---|---|
| Salts | 0 | 14.4 | 1.39 |
| Citric acid | 44.4 | 49.5 | Reference |

Test B - pH - 1.7

| Component | Net retention Volume | Peak Width at 0.5 Height | Resolution (0.5 Height) |
|---|---|---|---|
| Salts | 0 | 11.6 | 1.49 |
| Citric acid | 42.2 | 45.1 | Reference |

Test C - pH - 0.9

| Component | Net retention Volume | Peak Width at 0.5 Height | Resolution (0.5 Height) |
|---|---|---|---|
| Salts | 0 | 13.3 | 1.4 |
| Citric acid | 40.9 | 45.1 | Reference |

The results are also shown in Figure 3A in which it is clear that while citric acid is more strongly adsorbed than the other components, there is a substantial loss of citric acid which is unadsorbed and removed with the salts and carbohydrates (not shown). Citric acid is satisfactorily separated in the process in Figure 3B where the results are judged good and in Figure 3C where the results are judged excellent. The process clearly will have commercial feasibility at a pH of 1.7 and lower. At a pH of2.4 (Figure 3A), however, it is noted that a substantial amount of the citric acid will be recovered in the form of the citrate, $H_2CA^{-1}$, in the raffinate with the salts and carbohydrates. From this, it is evident that the ionized, soluble species should be reduced, as explained previously, by maintaining a lower pH in the feed, thereby driving the equilibrium in Equation 1 to the left.

EXAMPLE II

This example presents the results of using a neutral crosslinked styrene divinylbenzene (XAD-4) and a neutral crosslinked polyacrylic ester copolymer (XAD-8) with the same separation feed mixture as Example I at different pHs to demonstrate the poor separation when the pH is 2.4 or higher, or above the first ionization constant, $pKa_1$= 3.13, of citric acid. The same procedure and apparatus previously described in Example I were used in the separation, except the temperature was 60°C and 5 ml of feed mixture was used.

Figures 4A, 4B and 4C are, respectively, graphical presentations of the results of the pulse tests using XAD-4 at pHs, respectively, of 2.4, 1.7 and 0.9. Figure 4A shows that citric acid "breaks through" with the salts (and carbohydrates). This problem can be partially alleviated by lowering the pH to 1.7 as in Figure 4B. An excellent separation can be achieved by lowering the pH further to 0.9 as in Figure 4C. This separation, with adjustment of the pH, again, clearly has commercial utility.

Figures 4D and 4E are, respectively, graphical representations of the results of pulse tests, run under the same conditions as above, using XAD-8 at pHs of 2.8 and 1.4 and temperatures of 65°C. Figure 4D, which was made at pH of 2.8, shows no separation, but rather the salts, carbohydrates and citric acid eluting together initially. After about 67 ml, after most of the carbohydrates and salts and some of the citric acid have been recovered, some relatively pure citric acid can be obtained, but recovery is low. Figure 4E, which was made at a pH of 1.4, shows a selectivity between citric acid and carbohydrates and salts which results in a satisfactory separation and recovery of the citric acid.

EXAMPLE III

This example presents the results of using a neutral crosslinked styrene divinylbenzene copolymer (XAD-4) with the same separation feed mixture as Example I at two different pHs to demonstrate the poor separation when the pH is 2.4 or higher. The same procedure and apparatus previously described in Example I were used, except the temperature was 93°C in Figures 5A and 5B and the amount of feed mixture was 10 ml.

Figures 5A and 5B are, respectively, graphical presentations of the results of pulse tests using XAD-4 at pHs, respectively, of 2.8 and 1.4. Figure 5A shows that citric acid "breaks through" with the salts and carbohydrates. This problem can be alleviated by lowering the pH to 1.4 as in Figure 5B. This separation, with adjustment of the pH, again, clearly has commercial utility.

EXAMPLE IV

The procedure and apparatus previously described in Example I was used on the samples of this example. The temperature was 60°C and 5 ml of feed mixture was used. The feed composition was similar to that previously used except that citric acid has been concentrated to 40% in the feed mixture. The effect of concentration on the pH will be seen. In Figure 6A, even with the temperature at 60°C, the pH of 1.9 is too high to separate the citric acid at 40% concentration. By adjusting the pH downward as in Figures 6B and 6C, the citric acid is preferentially adsorbed and excellent separation is achieved at a pH of 1.13 and at a pH of 0.5. In each of these samples, carbohydrates were not analyzed, but it can be assumed that the carbohydrates closely followed the salts in the separation.

EXAMPLE V

The procedure and apparatus previously described in Example I was used on the three samples of this example. The temperature was 93°C and the amount of feed mixture was 5 ml. The feed composition was similar to that previously used except that citric acid has been concentrated to 40% in the feed mixture to demonstrate the further effect of concentration on the pH. In Figure 7A, even with the temperature at 93°C, the pH of 1.8 is too high to separate the citric acid at 40% concentration. By adjusting the pH downward as in Figures 7B and 7C, the citric acid is preferentially adsorbed and excellent separation is achieved. Again, carbohydrates were not analyzed, but it can be assumed that the carbohydrates closely followed the salts in the separation.

EXAMPLE VI

The pulse test of Example I was repeated on two 50% citric acid samples using XAD-4 adsorbent. The desorbent in both cases was water. The composition of the feed used was the same as used in Example I except that citric acid has been concentrated to 50%. The temperature was 93°C. In the first sample, the pH was 1.5. As shown in Figure 8A, citric acid was not separated. After reducing the pH to 1.0 in the second sample, citric acid was readily separated as seen in Figure 8B. Again, carbohydrates were not analyzed, but assumed to closely follow the salts. The separation in Figure 8B was judged good.

EXAMPLE VII

The separation example represented by Figures 7B and 7C required high temperatures, e.g., 93°C to achieve the separation of 40% citric acid due to the difficulty in desorbing citric acid from the XAD-4 adsorbent. In this example, high temperatures, which adversely affect the adsorbent life and the cost to operate, are eliminated and the separation is readily achieved at 45°C through the use of a desorbent mixture of 10% (by wt.) acetone and 90% water. Referring to Figure 9, a feed comprising 40% (wt.) citric acid, 4% carbohydrates and 2% salts of the following elements: $K^+$, $Na^+$, $Mg^{++}$, $Fe^{+++}$, $Ca^{++}$ plus proteins and amino acids, was introduced into the pulse test apparatus as set forth previously and the test

ran as before except that the temperature was 45°C. In this test, the pH was maintained at 0.5, but the desorbent contained acetone as mentioned above. The net retention volume for citric acid was 10.7 ml, and the resolution was 0.61 and, therefore, the separation was easily made.

EXAMPLE VIII

In this example, four pulse tests were run with a weakly basic anion exchange resin having a tertiary amine function hydrogen bonded to a sulfate ion, in a cross-linked gel-type acrylic resin matrix (AG4-X4 made by Bio Rad Laboratories, Richmond, California) having a tertiary amine function hydrogen bonded to a sulfate ion, in a cross-linked acrylic resin matrix to determine the ability of the adsorbent to separate citric acid from its fermentation mixture of carbohydrates (DP1, DP2, DP3, including glucose, xylose, arabinose and raffinose) and ions of salts, including$Na^+$, $K^+$, $Mg^{++}$, $Ca^{++}$,$Fe^{+++}$, $Cl^-$, $SO_4^=$, $PO_4^=$ and $NO_3^-$, amino acids and proteins at a pH of 1.6. The first test was run at a temperature of 75°C. The remaining tests were run at 60°C. Citric acid was desorbed with water in Pulse Test No. 1 (Figure 10) and sulfuric acid in two concentrations: 0.05 (Pulse Test No. 2) and 0.25N (Pulse Test No. 3). Pulse Test No. 4 was like Pulse Test No. 2 except that it was made after the adsorbent was used with 24 bed volumes of feed. The fermentation feed mixture had the following composition:

| Feed Composition | Per Cent |
|---|---|
| Citric Acid | 40% |
| Salts ($K^+$, $Na^+$, $Ca^{++}$, $Mg^{++}$ $Fe^{+++}$) | 1.5% |
| Carbohydrates (Sugars) | 4% |
| Others ($SO_4^=$, $Cl^-$, $PO_4^=$, $NO_3^-$, proteins and amino acids) | 5% |
| Water | 49.5% |

Retention volumes and separation factor (β) were obtained using the pulse test apparatus and procedure previously described in Example I except that a 5cc sample was used. The net retention volume (NRV) for the citric acid was calculated by measuring the distance from the midpoint of the salt envelope as the reference point to the midpoint of the citric acid envelope. The separation factor, β, is calculated from the ratio of the retention volumes of the components to be separated to the retention volume for the first salt componet (i.e. Salts 1).

The results for these pulse tests are shown in the following Table No. 5.

TABLE NO. 5

| Pulse Test | Resin/Desorbent | Feed Component | NRV | B |
|---|---|---|---|---|
| 1 | AG4-X4/Water | Salts 1 | 1.6 | 34.25 |
| | | Citric Acid | 54.8 | Reference |
| | | Unknowns A | 0 | Tracer |
| | | Unknowns B | 6.6 | 8.30 |
| | | Salts 2 | 54.6 | 1.00 |
| 2 | AG4-X4/0.05N $H_2SO_4$ | Salts | 3.2 | 11.87 |
| | | Citric Acid | 38.0 | Reference |
| | | Unknown A | 0 | Tracer |
| | | Unknown B | 2.7 | 14.07 |
| 3 | AG4-X4/0.25N $H_2SO_4$ | Unknowns A | 0 | Tracer |
| | | Citric Acid | 26.9 | Reference |
| | | Salts | 2.3 | 11.70 |
| | | Unknowns B | 7.6 | 3.54 |
| 4 | AG4-X4/0.05N $H_2SO_4$ | Unknowns A | 0 | Tracer |
| | | Citric Acid | 38.0 | Reference |
| | | Salts | 2.4 | 15.8 |
| | | Unknowns B | 7.2 | 5.28 |

The results of Pulse Tests 2-4 are similar to Figure 10. From Table 5, it is clear that while citric acid is satisfactorily separated in the process, in highly purified form, with water, desorption with water is slower than with dilute sulfuric acid as evidenced by larger net retention volume. After aging the adsorbent with 24 bed volumes of feed, the adsorbent shows no signs of deactivation, as observed in Figure 13, which is substantially identical to Figure 11 (conducted under identical conditions with fresh adsorbent).

EXAMPLE IX

The first pulse test of Example VIII was repeated using the same procedure and apparatus except that the temperature was 65°C. The desorbent was water. This example presents the results of using a macroporous weakly basic anionic exchange resin possessing a cross-linked polystyrene matrix (Dowex 66) with the same separation feed mixture as Example VIII (40% citric acid) in the first two pulse tests at a pH of 7.0 and 3.5 (Figures 11A and 11B, respectively) to demonstrate the failure to accomplish the desired separation when the pH is above the first ionization constant, $pKa_1 = 3.13$, of citric acid, and more specifically in these two samples, where the concentration of citric acid is 40%, when the pH is above 1.7. In the third part of the example (represented by Figure 11C), the feed was diluted to 13% citric acid and the pH reduced to 2.4. While there is evident improvement, it is apparent that the pH and/or concentration will have to be reduced further to prevent "breakout" of the citric acid. For example, at 13% concentration, it is estimated that the pH must be lowered to about 1.6 to 2.2

Figures 11A and 11B are, respectively, graphical presentation of the results of the pulse test using Dowex 66 at pHs, respectively, of 7.0 and 3.5. Figures 11A and 11B show that citric acid "breaks through" with the salts (and carbohydrates) at the higher pHs. This problem can be partially alleviated by reducing the concentration to 13% and lowering the pH to 2.4 as in Figure 11C, where it is shown that only a small amount of citric acid is not adsorbed and "breaks through" in the raffinate while most is adsorbed onto the adsorbent resin (but not desorbed in this Figure). This separation, with adjustment of the concentration and pH to optimum levels, clearly will have commercial utility.

EXAMPLE X

Three additional pulse tests under the same conditions as Example VIII, except as noted, were made on citric acid samples of the same feed composition, but with two different adsorbents. The desorbent in the first two samples was 0.05 N $H_2SO_4$ (Figures 12 and 13A) while water was used in the third sample

(Figure 13B). The composition of the feed used was the same as used in Example VIII. The temperature was 60°C and the pH was 1.6. The adsorbent #1 in the first test was a macroporous pyridine function-containing divinylbenzene cross-linked resin of the following formula:

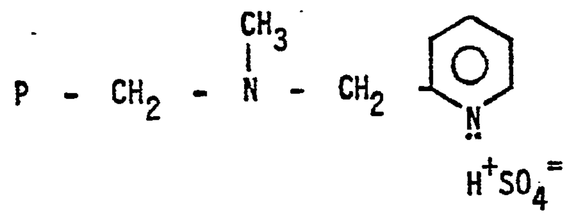

where P is the polystyrene moiety forming the resin. The second adsorbent (#2), used in the second and third samples, is a tertiary amine, also with a pyridine functional group, having the following formula:

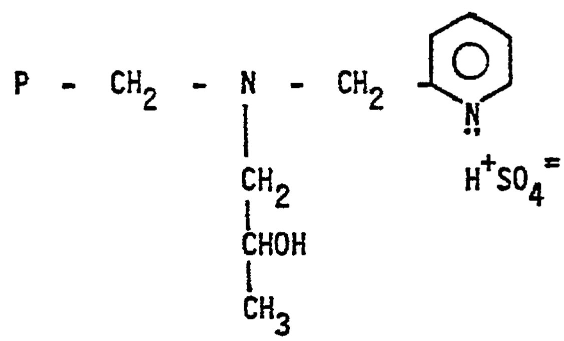

where P is as defined above. Both resins are cross-linked with divinylbenzene. In some cases, while water is an effective desorbent, with excellent separation, it is not strong enough to recover the adsorbed citric acid quickly enough to make the process commercially attractive. See Figure 13B, in which the conditions are the same as above, using adsorbent #2, where water is the desorbent. In this case citric acid does not elute until about 95 ml of desorbent have passed through the adsorbent. Dilute sulfuric acid is, therefore, the preferred desorbent, as will be apparent from the results shown in Figures 12 and 13A. Also, from Figures 12, 13A and 13B, it will be seen that an excellent separation of citric acid is obtained.

EXAMPLE XI

The procedure, conditions and apparatus previously described in Example VIII were used to separate four samples of citric acid from the same feed with two different resins of the same class of adsorbent as Example VIII, (except that in the first and fourth samples, the column temperature was 50°C and the desorbent was 0.05N $H_2SO_4$; in the second and third samples the pH was 2.2 and the desorbent was dilute sulfuric acid at 0.15 N concentration). Both resins, IRA-68 and IRA-35, obtained from Rohm and Haas, have an amine function and the following structural formula:

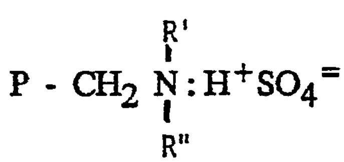

where P is the polyacrylic matrix, and
R′ and R″ = $CH_3$.
Amberlite IRA-68 (Sample Nos. 1, 2 and 3) is a gel-type resin. IRA-35 (Sample No. 4) is a macroreticular-type resin. Sample No. 3 was identical to Sample No. 2, except that the adsorbent had previously been used to separate 69 bed columns of the feed. Sample Nos. 1 and 2 are both excellent adsorbents for separating citric acid from its fermentation broth within the pH range of 1.6 to 2.2. Sample No. 3, after aging the adsorbent with 69 bed volumes of feed, demonstrates the stability of the resin (little or no deactivation has taken place) in this separation. Net retention volume (NRV) and selectivity (β) are shown in the following Table 6.

TABLE 6

| Sample No. | Resin | Component | NRV | B |
|---|---|---|---|---|
| 1 | Amberlite IRA-68 | Salts | 5.5 | 8.24 |
| | | Citric Acid | 45.3 | Reference |
| | | Unknown A | 0 | Tracer |
| | | Unknown B | 9.3 | 4.87 |
| 2 | Amberlite IRA-68 | Salts | 2.3 | 12.61 |
| | | Citric Acid | 29.0 | Reference |
| | | Unknown A | 0 | Tracer |
| | | Unknown B | 6.5 | 4.46 |
| 3 | Amberlite IRA-68 | Salts | 2.85 | 10.32 |
| | | Citric Acid | 29.4 | Reference |
| | | Unknown A | 0 | Tracer |
| | | Unknown B | 7.0 | 4.2 |
| 4 | Amberlite IRA-35 | Salts | 1.3 | 27.38 |
| | | Citric Acid | 36.9 | Reference |
| | | Unknown A | 0 | Tracer |
| | | Unknown B | 5.9 | 6.25 |

In a further comparison of the claimed adsorbents of Examples I through VII with Examples VIII through XI, several samples of the extract were analyzed for readily carbonizable impurities (RCS) (Food & Chemical Codex (FCC) Monograph #3) and potassium level. RCS is determined in the following manner: a 1 gm sample of the extract (actual concentration of citric acid is determined) is carbonized at 90°C with 10 ml of 95% $H_2SO_4$. The carbonized substance is spectrophatometrically measured at 500 nm using a 2-cm cell with a 0.5 inch diameter tube and the amount of RCS is calculated for 50% citric acid solution. The number arrived at can be compared with that obtained by using this procedure on the cobalt standard solution of the FCC test mentioned above. Potassium is determined by atomic adsorption spectroscopy. For comparison, the same analytical determinations were made on a sample of the same feed and RCS calculated for 50% citric acid with XAD-4, AG4-X4, and adsorbents No. 1 and No. 2 of Example III. The results are shown in Table 7.

TABLE 7

EXTRACT QUALITY (RCS/POTASSIUM) BY PULSE TEST

| Adsorbent | Desorbent | RCS Calculated (for 50% C.A.) | ppmK | C.A. Net Ret. Vol. |
|---|---|---|---|---|
| XAD-4 | $H_2O$ | 6.86, 8.98 | 59, 137 | 13.0 |
| AG4-X4 | .05N.$H_2SO_4$ | 1.77, 1.42 | 24, 81 | 34.8 |
| #2 (Ex. X) | .05N.$H_2SO_4$ | 3.17, 3.33 | 24, 54 | 30.8 |
| #1 (Ex. X) | .05N.$H_2SO_4$ | 2.17 | 62 | 31.0 |

An improvement in both reduction of levels of RCS and K for the weakly basic resins compared to the neutral resins is indicated by this data. In all samples, RCS was reduced by at least 50% and in two samples, K was reduced by over 50%. It is noted from the net retention volume that both classes of adsorbents have good resolution, but the strong base adsorbents suffer somewhat from increased cycle times. The cycle times can be reduced by using higher concentrations of sulfuric acid, e.g., up to about 0.2N, in the preferred range of 0.1 to 0.2N.

In another embodiment, citric acid adsorbed on the adsorbent may be converted in situ to a citrate before being desorbed, for example, by reaction with an alkaline earth metal or alkali metal hydroxide or ammonium hydroxide and then immediately eluted using a metal hydroxide, ammonium hydroxide or water as the desorbent. Deactivation of the adsorbent by the unknown impurities may take place in time, but the adsorbent may be regenerated by flushing with a stronger desorbent, e.g., a higher concentration of sulfuric acid than the desorbent, an alkali metal hydroxide or $NH_4OH$, or an organic solvent, e.g., acetone or alcohol.

EXAMPLE XII

In this example, two pulse tests were run with a gel-type strongly basic anion exchange resin (IRA 458 made by Rohm & Haas Co.) having the structural formula like (1) on page 21 above, substituted with three methyl groups, to determine the ability of the adsorbent to separate citric acid from its fermentation mixture of carbohydrates (DP1, DP2, DP3, including glucose, xylose, arabinose and raffinose) and ions of salts, including$Na^+$, $K^+$, $Mg^{++}$, $Ca^{++}$,$Fe^{+++}$, $Cl^-$, $SO_4^=$, $PO_4^\equiv$ and $NO_3^-$, amino acids and proteins at a pH of 2.2. P is acrylic cross-linked with divinylbenzene. Pulse test Sample No. 1 was run at a temperature of 50°C. Pulse test Sample No. 2 was run at 60°C, but after the bed had been aged with 33 bed volumes of feed. Further runs to 62 bed volumes have been made with no signs of deactivation of the adsorbent. Citric acid was desorbed with 0.1N solution of sulfuric acid in both samples. The fermentation feed mixture had the following composition:

| Feed Composition | Per Cent |
|---|---|
| Citric Acid | 40 |
| Salts ($K^+$, $Na^+$, $Ca^{++}$, $Mg^{++}$ $Fe^{+++}$) | 1.5 |
| Carbohydrates (Sugars) | 4 |
| Others ($SO_4^=$, $Cl^-$, $PO_4^=$, $NO_3^-$, proteins and amino acids) | 5 |
| Water | 49.5 |

Retention volumes and separation factor were obtained using the pulse test apparatus and procedure previously described in Example I.

The results for these pulse tests are shown in the following Table No. 8.

TABLE NO. 8

| Sample No. | Resin | Feed Component | NRV | B |
|---|---|---|---|---|
| 1 | IRA-458 | Salts | 1.0 | 38.9 |
| | | Citric Acid | 38.9 | Reference |
| | | Unknowns A | 0 | Tracer |
| | | Unknowns B | 6.6 | 5.89 |
| 2 | IRA-458 | Salts | 0.9 | 43.3 |
| | | Citric Acid | 39.0 | Reference |
| | | Unknown A | 0 | Tracer |
| | | Unknown B | 7.1 | 5.49 |

It is clear that citric acid is satisfactorily separated in the process, and after aging the adsorbent with 33 bed volumes of feed, the adsorbent shows no signs of deactivation, which is substantially identical to the results under closely identical conditions with fresh adsorbent.

EXAMPLE XIII

The pulse test of Example XII was repeated on additional citric acid samples using the same feed, but a different, macroporous, strongly basic anionic exchange adsorbents, IRA-958, possessing quaternary ammonium functions and an acrylic resin matrix cross-linked with divinylbenzene matrix. The desorbent was 0.05 N $H_2SO_4$. The composition of the feed used was the same as used in Example XII. The temperature was 60°C and the pH was 1.6. The adsorbent in this test was a resin obtained from Rohm & Haas having the structure (1) shown on page 21, where R is methyl.

As shown in Figure 14, citric acid starts eluting after 45 ml of desorbent have passed through the adsorbent and is very effectively separated from the fermentation mixture in high purity with excellent recovery.

EXAMPLE XIV

The pulse test of Example XII was repeated on an additional citric acid sample using the same feed, but a different, strongly basic anionic exchange resin adsorbent, AG2-X8 (obtained from Bio Rad Company) having a structure like formula (2) above, (page 21) where R is methyl, with a cross-linked polystyrene gel-type resin matrix having quaternary ammonium functional groups thereon. The desorbent was 0.15N $H_2SO_4$. The composition of the feed used was the same as used in Example XI. The temperature was 50°C and the pH was 2.2.

As shown in Figure 15, citric acid starts eluting at about 43 ml of desorbent have passed through the adsorbent and is very effectively separated from the fermentation mixture in high purity with excellent recovery.

In a further comparison of adsorbents of Examples I through VII with Examples XII through XIV, several samples of the extract were analyzed for readily carbonizable impurities (RCS) (Food & Chemical Codex (FCC) Monograph #3) and potassium level as described above. The results for each of the adsorbents, XAD-4, IRA 458, IRA 959 and AG2-X4 with the indicated desorbent are shown in the following Table 9.

TABLE 9

EXTRACT QUALITY (RCS/POTASSIUM) BY PULSE TEST

| Adsorbent | Desorbent | RCS (Calculated at 50% CA) (Units) | ppmK (Calculated at 50% C.A.) | CA Net Retention Volume |
|---|---|---|---|---|
| XAD-4 | $H_2O$ | 6.86<br>8.98 | 59<br>137 | 13.0 |
| IRA 458 | 0.1N $H_2SO_4$ | 1.5 | 80 | 37.9 |
| IRA 958 | 0.05N $H_2SO_4$ | 2.73 | 82 | 32 |
| AG2-X4 | 0.15N $H_2SO_4$ | 5.3 | 131 | 43 |

An improvement in both reduction of levels of RCS and K for the strongly basic resins compared to the neutral resins is indicated by this data. In all samples, RCS was reduced by between 40-85% and K was reduced between 0-20%. It is noted from Examples XII, XIII and XIV (Fig. 14), that both classes of adsorbents have good separation, but the present adsorbents suffer somewhat from increased cycle times. The cycle times can be reduced by using higher concentrations of sulfuric acid, e.g., up to about 0.2N in the preferred range of 0.1 to 0.2N.

In another embodiment, citric acid, adsorbed on the adsorbent may be converted in situ to a citrate before being desorbed, for example, by reaction with an alkaline earth metal hydroxide, alkali metal hydroxide or ammonium hydroxide and then immediately eluted using a metal hydroxide, ammonium hydroxide or water, as the desorbent. Deactivation of the adsorbent by the unknown impurities may take place in time, but the adsorbent may be regenerated by flushing with a stronger desorbent, e.g., a high concentration of sulfuric acid than the desorbent, an alkali metal hydroxide or $NH_4OH$, or an organic solvent, e.g., acetone or alcohol.

**Claims**

1. A process for separating citric acid from a fermentation broth feed mixture containing citric acid characterized in that said mixture is contacted with a polymeric adsorbent selected from a neutral, crosslinked polystyrene polymer, a nonionic hydrophobic polyacrylic ester polymer, a weakly basic anionic exchange resin possessing tertiary amine or pyridine functional groups, and a strongly basic anionic exchange resin possessing quaternary amine functional groups, and mixtures thereof at adsorption conditions whereby selectively to adsorb said citric acid, and thereafter said citric acid is recovered from said adsorbent with a desorbent at desorption conditions.

2. A process according to claim 1 characterized in that said adsorption and desorption conditions include a temperature of from 20 to 200°C and a pressure of from atmospheric to 500 psig (3450 kPa gauge).

3. A process according to claim 1 or 2 characterized in that said desorption is effected in the liquid phase with water or an aqueous inorganic acid or a ketone or mixtures thereof.

4. A process according to any one of claims 1 to 3 characterized in that said absorbent comprises a tertiary amine functional group supported on a matrix comprising a crosslinked acrylic resin.

5. A process according to any one of Claims 1 to 3 characterized in that said adsorbent comprises a pyridine functional group supported on a matrix comprising a crosslinked polystyrene resin.

6. A process according to any one of Claims 1 to 3 characterized in that said adsorbent comprises a quaternary amine functional group supported on a matrix comprising a crosslinked acrylic resin.

7. A process according to any one of Claims 1 to 3 characterized in that said adsorbent comprises a quaternary ammonium salt of pyridine supported on a matrix comprising a crosslinked polystyrene resin.

8. A process according to any one of Claims 1 to 3, 6 and 7 characterised in that the quaternary ammonium group is in the sulphate form.

9. A process according to any one of claims 1 to 8 characterized in that it comprises the steps of:

(a) maintaining net fluid flow in a single direction, through a column of said adsorbent containing at least three zones having separate operational functions occurring therein and being serially interconnected, with the terminal zones of said column connected to provide a continuous connection of said zones;
(b) maintaining in said column an adsorption zone, defined by the adsorbent located between a feed input stream at an upstream boundary of said zone and a raffinate output stream at a downstream boundary of said zone;
(c) maintaining immediately upstream from said adsorption zone, a purification zone defined by the adsorbent located between an extract output stream at an upstream boundary of said purification zone and said feed input stream at a downstream boundary of said purification zone;
(d) maintaining immediately upstream from said purification zone, a desorption zone defined by the adsorbent located between a desorbent input stream at an upstream boundary of said zone and said extract output stream at a downstream boundary of said zone;
(e) passing said feed mixture into said adsorption zone at adsorption conditions to effect the selective adsorption of said citric acid by said adsorbent in said adsorption zone and withdrawing a raffinate output stream comprising the nonadsorbent components of said fermentation broth from said adsorption zone;
(f) passing the desorbent material into said desorption zone at desorption conditions to effect the displacement of said citric acid from the adsorbent in said desorption zone;
(g) withdrawing an extract output stream comprising said citric acid and desorbent material from said desorption zone;
(h) passing at least a portion of said extract output stream to a separation means and therein separating at separation conditions at least a portion of said desorbent material; and, (i) periodically advancing through said column of adsorbent in a downstream direction with respect to fluid flow in said adsorption zone, the feed input stream, raffinate output stream, desorbent input stream, and extract output stream to effect the shifting of zones through said adsorbent and the production of extract output and raffinate output streams.

10. A process according to Claim 9 characterized in that a buffer zone is maintained immediately upstream from said desorption zone, said buffer zone defined as the adsorbent located between the desorbent input stream at a downstream boundary of said buffer zone and the raffinate output stream at an upstream boundary of said buffer zone.

11. An adsorption process for separating citric acid from a fermentation broth feed mixture containing citric acid characterized in that said mixture is contacted at a pH lower than the first ionization constant $PKa_1$ of citric acid, with a weakly basic anionic exchange resin possessing tertiary amine or pyridine functional groups, at adsorption conditions whereby selectively to adsorb said citric acid, converting the citric acid into a salt by reaction with an alkaline earth metal hydroxide, alkali metal hydroxide or ammonium hydroxide, and eluting the salt with a metal hydroxide, ammonium hydroxide or water as eluant.

## Revendications

1. Procédé d'adsorption pour la séparation de l'acide citrique d'un mélange d'alimentation à base d'un bouillon de fermentation contenant de l'acide citrique, caractérisé en ce que ce mélange est mis contact à un pH inférieur à la première constante d'ionisation ($pKa_1l$) de l'acide citrique, avec un adsorbant polymère choisi parmi un polymère de type polystyrène neutre reticulé, un polymère non ionique hydrophobe de type polyester acrylique, une résine échangeuse d'anion faiblement basique possédant des groupes fonctionnels amine tertiaire ou pyridine, et une résine échangeuse d'anion fortement basique possédant des groupes fonctionnels amine quaternaire, et les mélanges de ceux-ci, dans des conditions d'adsorption appropriées pour adsorber sélectivement l'acide citrique, et l'acide citrique est ensuite récupéré à partir de l'adsorbant, à l'aide d'un desorbant dans des conditions de desorption.

2. Procédé selon la revendication 1, caractérisé en ce que les conditions d'adsorption et de desorption, comprennent une température de 20 à 200°C et une pression allant de la pression atmosphérique à une pression relative de 3450 kPa.

3. Procédé selon la revendication 1 ou 2, caractérisé en ce que la désorption est effectuée en phase liquide, avec de l'eau, un acide inorganique aqueux, une cétone ou un mélange de ceux-ci.

4. Procedé selon l'une quelconque des revendications 1 à 3, caractérisé en ce que l'adsorbant, comprend un groupe fonctionnel amine tertiaire fixé sur une matrice comprenant une résine acrylique réticulée.

5. Procédé selon l'une quelconque des revendications 1 à 3, caractérisé en ce que l'adsorbant, comprend un groupe fonctionnel pyridine fixé sur une matrice comprenant une résine de polystyrène reticulé.

6. Procédé selon l'une quelconque des revendications 1 à 3, caractérisé en ce que l'adsorbant, comprend un groupe fonctionnel amine quaternaire fixé sur une matrice comprenant une résine acrylique réticulée.

7. Procédé selon l'une quelconque des revendications 1 à 3, caracterisé en ce que l'adsorbant comprend un groupe salifié ammonium quaternaire derivé de la pyridine, fixé sur une matrice comprenant une résine de polystyrène réticulé.

8. Procédé selon l'une quelconque des revendications 1 à 3, 6 et 7, caractérisé en ce que le groupe ammonium quaternaire est sous forme sulfate.

9. Procédé selon l'une quelconque des revendications 1 à 8, caractérisé en ce qu'il comprend les étapes consistant:

(a) à maintenir un écoulement de fluide net dans une unique direction à travers une colonne de l'adsorbant, contenant au moins trois zones ayant des fonctions actives séparées et reliées en série, les zones terminales de la colonne étant raccordées de façon à relier de manière continue lesdites zones;

(b) à maintenir dans ladite colonne une zone d'adsorption, délimitee par l'adsorbant situé entre un courant d'alimentation entrant à l'extrémité amont de la zone et un courant de raffinat sortant à l'extrémité aval de la zone;

(c) à maintenir immédiatement en amont de la zone d'adsorption, une zone de purification délimitée par l'adsorbant situé entre un courant d'extrait sortant à l'extrémité amont de la zone de purification, et le courant d'alimentation entrant à l'extremité aval de la zone de purification;

(d) a maintenir immédiatement en amont de la zone de purification, une zone de désorption délimitée par l'adsorbant située entre un courant de désorbant entrant à l'extrémité amont de la zone, et le courant d'extrait sortant à l'extrémité aval de la zone;

(e) à faire passer le mélange d'alimentation, dans des conditions d'adsorption, à l'intérieur de la zone d'adsorption, afin d'effectuer l'adsorption sélective de l'acide citrique sur l'adsorbant dans la zone d'adsorption, et à retirer de la zone d'adsorption, un courant de raffinat sortant comprenant les composants non adsorbés du bouillon de fermentation;

(f) à faire passer dans des conditions de désorption, l'agent de désorption à l'interieur de la zone de désorption, afin de déplacer l'acide citrique dans l'adsorbant à l'intérieur de la zone de désorption;

(g) à retirer de la zone de désorption, un courant d'extrait sortant comprenant l'acide citrique et l'agent de désorption;

(h) à faire passer au moins une portion du courant d'extrait sortant, dans des moyens de séparation afin d'y séparer dans des conditions de séparation, au moins une portion de l'agent de désorption; et

(i) à faire avancer périodiquement dans la colonne d'adsorbant en direction aval par rapport à l'écoulement de fluide dans ladite zone d'adsorption, le courant d'alimentation entrant, le courant de raffinat sortant, le courant de désorbant entrant et le courant d'extrait sortant, afin de décaler les zones dans l'adsorbant et de produire des courants sortants d'extrait et de raffinat.

10. Procédé selon la revendication 9, caractérisé en ce qu'on maintient une zone tampon immédiatement en amont de la zone de désorption, la zone tampon étant delimitée par l'adsorbant situé entre le courant de désorbant entrant à l'extrémité aval de la zone tampon, et le courant de raffinat sortant à l'extrémité amont de la zone tampon.

11. Procédé d'adsorption pour séparer l'acide citrique d'un mélange d'alimentation à base d'un bouillon de fermentation contenant de l'acide citrique, caractérisé en ce que le mélange est mis en contact à un pH inférieur à celui de la première constante d'ionisation ($pKa_1$) de l'acide citrique, avec une résine échangeuse d'anion faiblement basique, possédant des groupes fonctionnels amine tertiaire ou pyridine, dans des conditions d'adsorption appropriées pour adsorber sélectivement l'acide citrique, en ce qu'on convertit l'acide citrique en un sel, par réaction avec un hydroxyde de métal alcalino-terreux, un hydroxyde de métal alcalin ou un hydroxyde d'ammonium, et en ce qu'on élue le sel avec un hydroxyde métallique, un hydroxyde d'ammonium ou de l'eau comme éluant.

## Patentansprüche

1. Adsorptionsverfahren zur Trennung von Zitronensäure von einem Zitronensäure enthaltenden Gärflüssigkeits-Zuflußgemisch, dadurch gekennzeichnet, daß das genannte Gemisch bei einem unter der ersten Dissoziationskonstanten ($pKa_1$) der Zitronensäure liegenden pH mit einem polymeren Adsorptionsmittel, das ausgewählt ist aus der Gruppe: neutrales vernetztes Polystyrolpolymer, nichtionogenes hydrophobes Polyacrylesterpolymer, schwach basisches Anionenaustauscherharz mit funktionellen tertiären Amin- oder Pyridin-Gruppen und stark basisches Anionenaustauscherharz mit funktionellen quaternären Amingruppe sowie Mischungen dieser Substanzen, unter Adsorptionsbedingungen in Kontakt gebracht wird, um dadurch die genannte Zitronensäure selektiv zu adsorbieren, und daß anschließend die genannte Zitronensäure aus dem Adsorptionsmittel unter Desorptionsbedingungen mit Hilfe eines Desorptionsmittels rückgewonnen wird.

2. Verfahren nach Anspruch 1, dadurch gekennzeichnet, daß die genannten Adsorptions- und Desorptionsbedingungen eine Temperatur von 20 bis 200°C und einen Druck von Atmosphärendruck bis 3450 kPa Überdruck (500 psig) bedeuten.

3. Verfahren nach Anspruch 1 oder 2, dadurch gekennzeichnet, dasßdie genannte Desorption in flüssiger Phase mit Wasser oder einer wäßrigen anorganischen Säure oder einem Keton oder Gemischen dieser Substanzen erfolgt.

4. Verfahren nach einem der Ansprüche 1 bis 3, dadurch gekennzeichnet, daß das genannte Adsorptionsmittel eine funktionelle tertiäre Amingruppe auf einer Matrix umfaßt, die ein vernetztes Acrylharz enthält.

5. Verfahren nach einem der Ansprüche 1 bis 3, dadurch gekennzeichnet, daß das genannte Adsorptionsmittel eine funktionelle Pyridingruppe auf einer Matrix umfaßt, die ein vernetztes Polystyrolharz enthält.

6. Verfahren nach einem der Ansprüche 1 bis 3, dadurch gekennzeichnet, daß das genannte Adsorptionsmittel eine funktionelle quaternäre Amingruppe auf einer Matrix umfaßt, die ein vernetztes Acrylharz enthält.

7. Verfahren nach einem der Ansprüche 1 bis 3, dadurch gekennzeichnet, daß das genannte Adsorptionsmittel ein quarternäres Pyridin-Ammoniumsalz auf einer Matrix umfaßt, die ein vernetztes Polystyrolharz enthält.

8. Verfahren nach einem der Ansprüche 1 bis 3, 6 und 7, dadurch gekennzeichnet, daß die quaternäre Ammoniumgruppe in Form des Sulfats vorliegt.

9. Verfahren nach einem der Ansprüche 1 bis 8, dadurch gekennzeichnet, daß es die folgenden Schritte umfaßt:

a) in einer einzigen Richtung eine Nettofluidströmung durch eine Säule des genannten Adsorptionsmittels hindurch aufrechterhalten, das mindestens drei Zonen aufweist, in denen getrennte Arbeitsfunktionen ablaufen und die hintereinanderliegend verbunden sind, wobei die Endzonen der genannten Säule verbunden sind, um eine fortlaufende Verknüpfung der genannten Zonen herbeizuführen;

b) in der genannten Säule eine Adsorptionszone aufrechterhalten, die definiert ist durch das Adsorptionsmittel, das sich zwischen einem Zuflußgemisch-Eingangsstrom an einer stromauf gelegenen Grenze der genannten Zone und einem Raffinat-Ausgangsstrom an einer stromab gelegenen Grenze der genannten Zone befindet;

c) unmittelbar stromauf von der genannten Adsorptionszone eine Reinigungszone aufrechterhalten, die definiert ist durch das Adsorptionsmittel, das sich zwischen einem Extrakt-Ausgangsstrom an einer stromauf gelegenen Grenze der genannten Reinigungszone und dem genannten Zuflußgemisch-Eingangsstrom an einer stromab gelegenen Grenze der genannten Reinigungszone befindet;

d) unmittelbar stromauf von der genannten Reinigungszone eine Desorptionszone aufrechterhalten, die definiert ist durch das Adsorptionsmittel, das sich zwischen einem Desorptionsmittel-Eingangsstrom an einer stromauf gelegenen Grenze der genannten Zone und dem genannten Extrakt-Ausgangsstrom an einer stromab gelegenen Grenze der genannten Zone befindet;

e) das genannte Zuflußgemisch in die genannte Adsorptionszone unter Adsorptionsbedingungen einleiten, um die selektive Adsorption der genannten Zitronensäure durch das genannte Adsorptionsmittel in der genannten Adsorptionszone zu bewirken, und einen Raffinat-Ausgangsstrom abziehen, der die nichtadsorbierbaren Bestandteile der genannten Gärflüssigkeit von der genannten Adsorptionszone umfaßt;

f) das Desorptionsmaterial in die genannte Desorptionszone unter Desorptionsbedingungen einleiten, um die Verdrängung der genannten Zitronensäure von dem Adsorptionsmittel in der genannten Desorptionszone herbeizuführen;

g) einen die genannte Zitronensäure und das Desorptionsmaterial umfassenden Extrakt-Ausgangsstrom aus der genannten Desorptionszone abziehen;

h) mindestens einen Teil des genannten Extrakt-Ausgangsstroms in eine Trenneinrichtung einleiten und mindestens einen Teil des genannten Desorptionsmittelmaterials darin unter Trennbedingungen abtrennen; und

i) durch die genannte Adsorptionsmittelsäule hindurch in Richtung stromab bezüglich des Fluidstroms in der genannten Adsorptionszone den Zufluß-Eingangsstrom, den Raffinat-Ausgangsstrom, den Desorptionsmittel-Eingangsstrom und den Extrakt-Ausgangsstrom periodisch vorwärts bewegen, um die Zonenverschiebung durch das genannte Adsorptionsmittel und die Herstellung von Extrakt-Ausgangsstrom und Raffinat-Ausgangsstrom herbeizuführen.

10. Verfahren nach Anspruch 9, dadurch gekennzeichnet, daß unmittelbar stromauf von der genannten Desorptionszone eine Pufferzone aufrechterhalten wird, die definiert ist als das Adsorptionsmittel, das sich zwischen dem Desorptionsmittel-Eingangsstrom an einer stromab gelegenen Grenze der genannten Pufferzone und dem Raffinat-Ausgangsstrom an einer stromauf gelegenen Grenze der genannten Pufferzone befindet.

11. Adsorptionsverfahren zur Trennung von Zitronensäure von einem Zitronensäure enthaltenden Gärflüssigkeits-Zuflußgemisch, dadurch gekennzeichnet, daß das genannte Gemisch bei einem unter der ersten Dissoziationskonstranten ($pKa_1$) von Zitronensäure liegenden pH mit einem schwach basischen Anionenaustauscherharz, das funktionelle tertiäre Amingruppen oder Pyridingruppen enthält, bei Adsorptionsbedingungen in Kontakt gebracht wird, um die genannte Zitronensäure zu adsorbieren, daß die Zitronensäure durch Reaktion mit einem Erdalkalimetallhydroxid, Alkalimetallhydroxid oder Ammoniumhydroxid in ein Salz überführt wird, und daß das Salz mit einem Metallhydroxid, einem Ammoniumhydroxid oder Wasser als Elutionsmittel eluiert wird.

FIG. 1



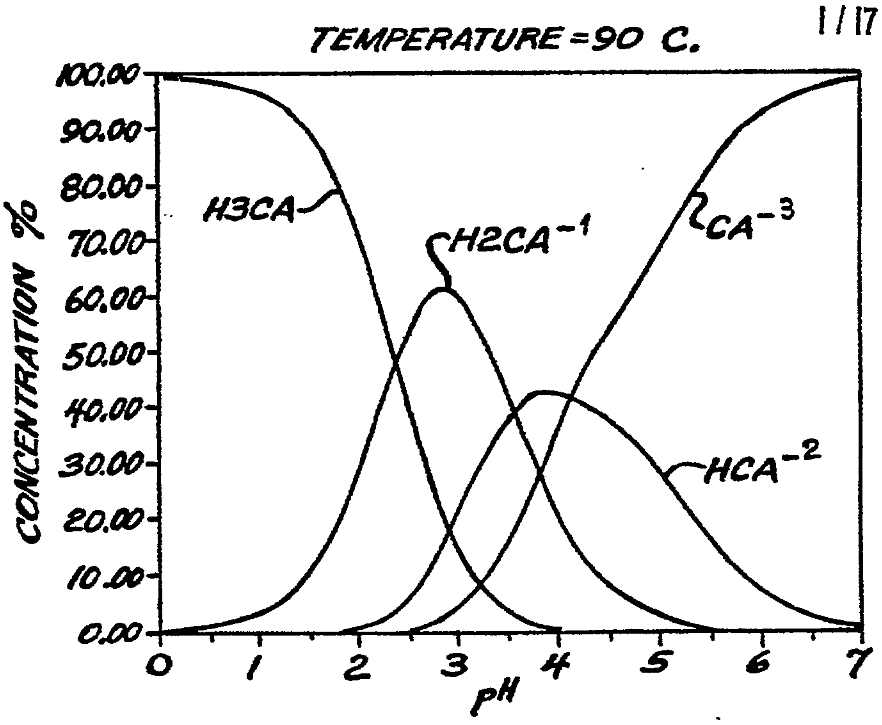


FIG. 2

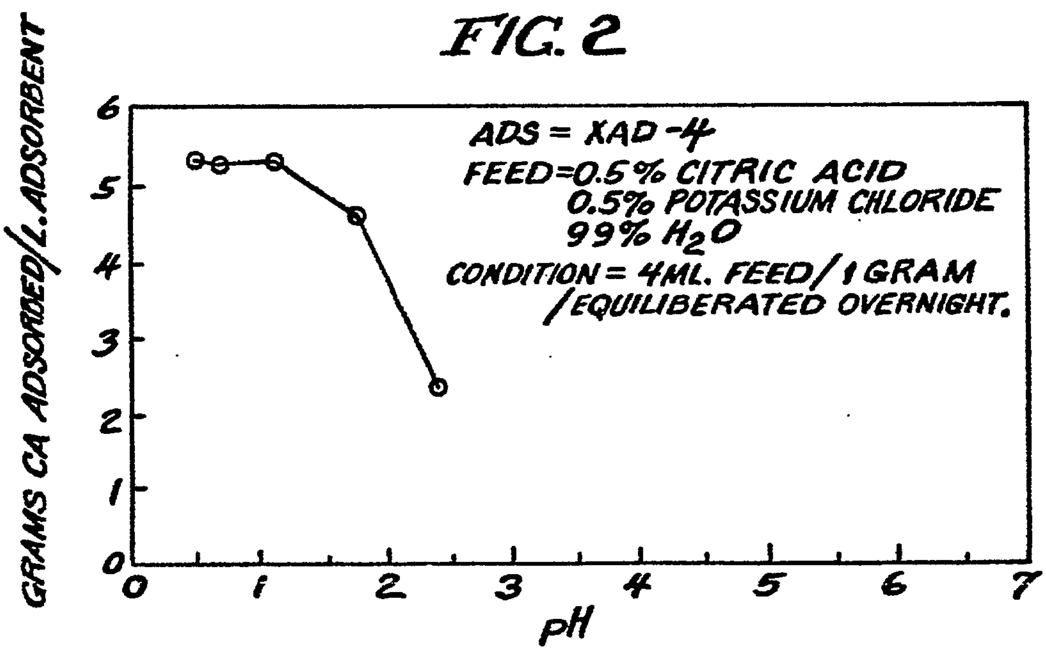


EFFECT OF pH ON CITRIC ACID ADSORPTION

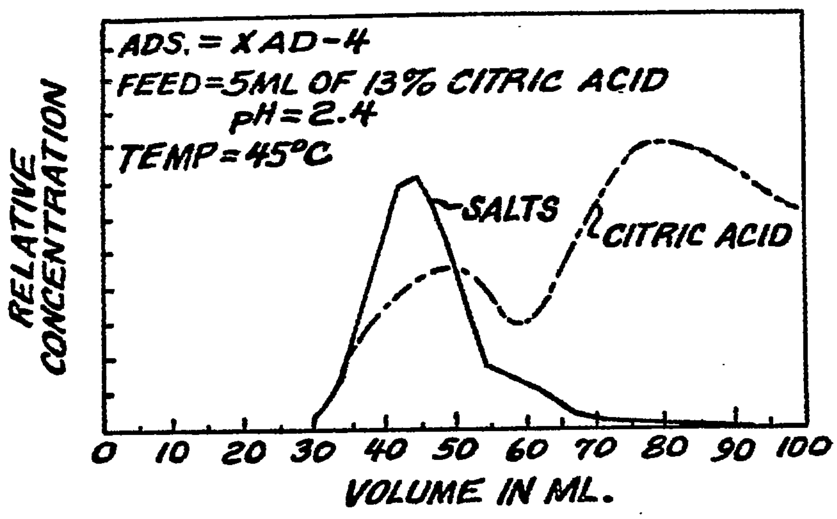


FIG.3A

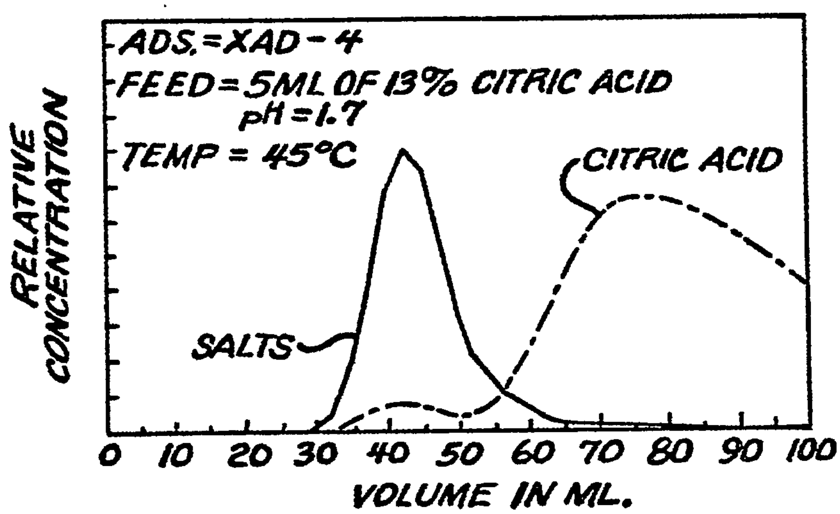


FIG.3B

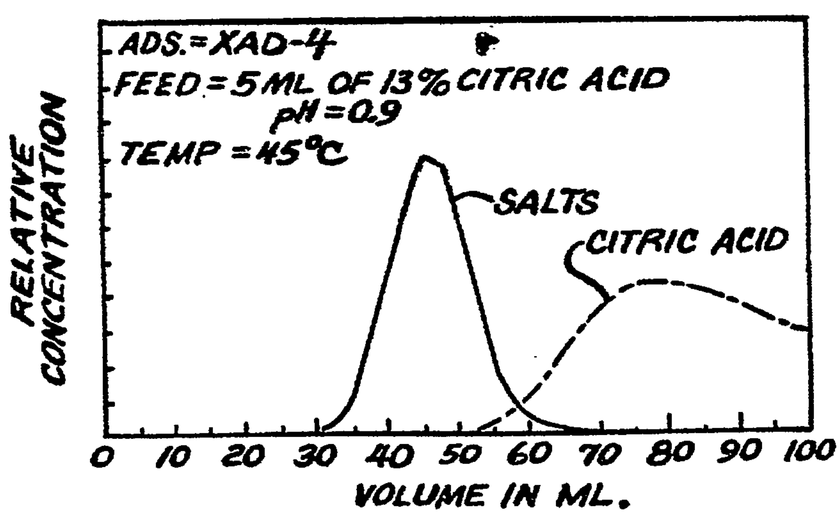


FIG.3C

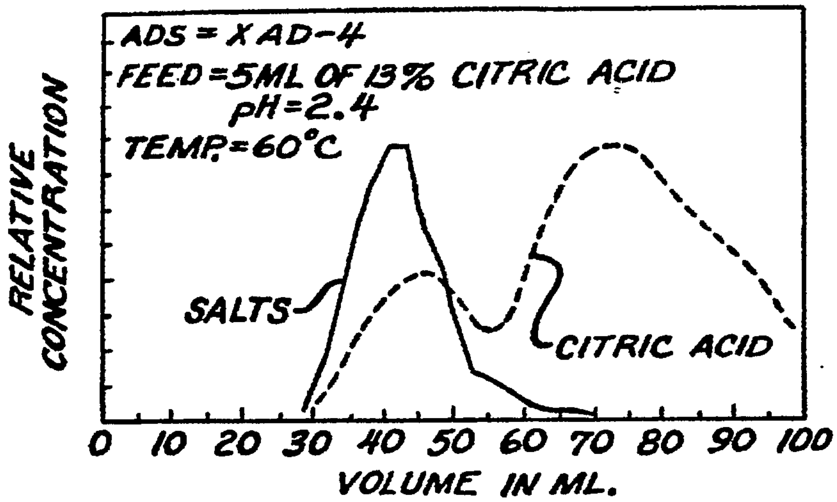


FIG. 4A

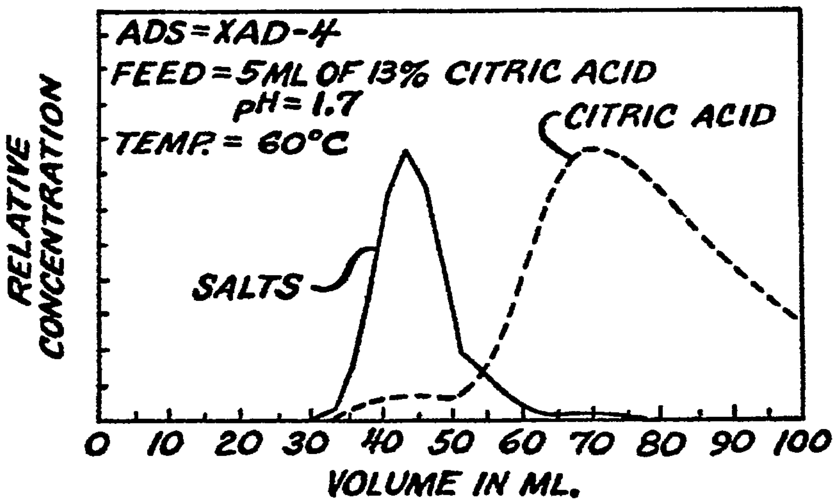


FIG. 4B

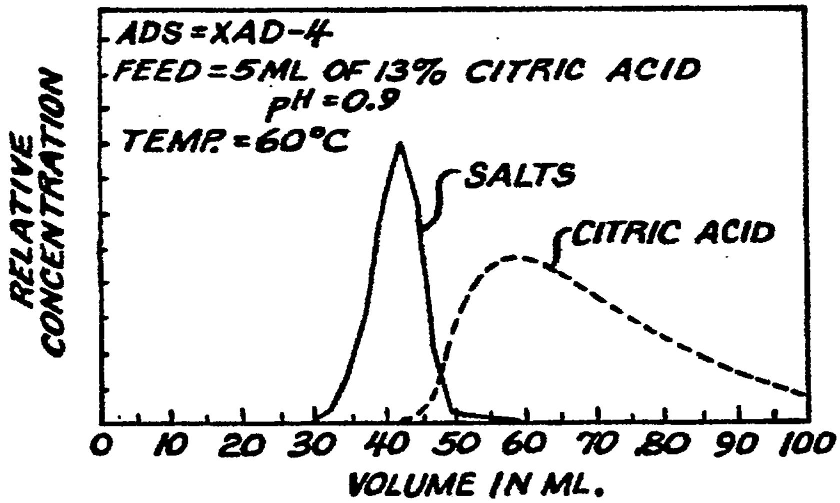


FIG. 4C

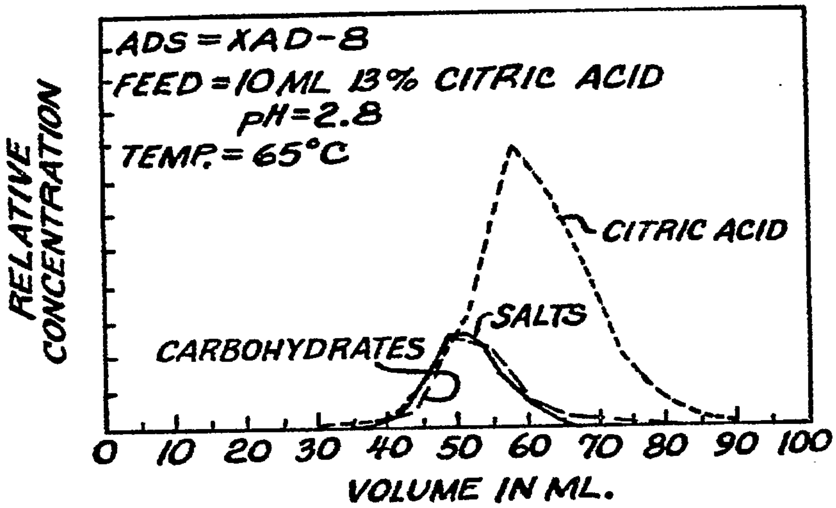
ADS = XAD-8
FEED = 10 ML 13% CITRIC ACID
pH = 2.8
TEMP. = 65°C
RELATIVE CONCENTRATION
CITRIC ACID
SALTS
CARBOHYDRATES
0 10 20 30 40 50 60 70 80 90 100
VOLUME IN ML.

FIG. 4D

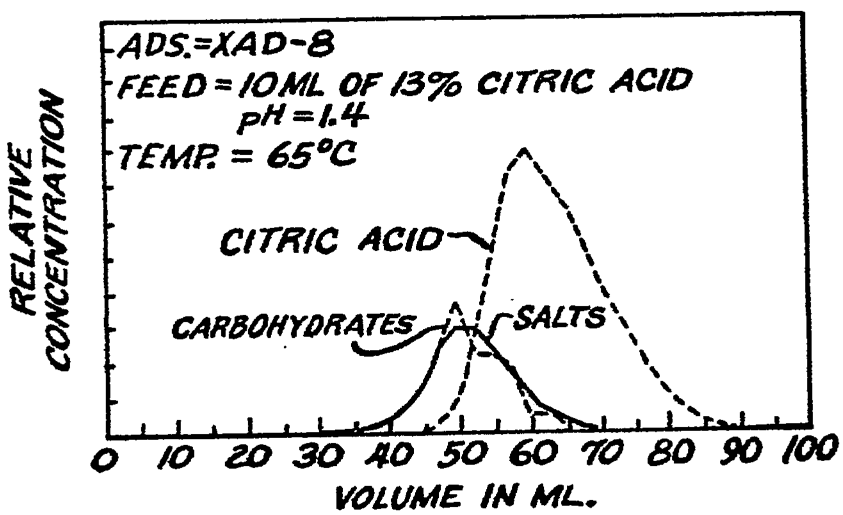
ADS. = XAD-8
FEED = 10 ML OF 13% CITRIC ACID
pH = 1.4
TEMP. = 65°C
RELATIVE CONCENTRATION
CITRIC ACID
CARBOHYDRATES
SALTS
0 10 20 30 40 50 60 70 80 90 100
VOLUME IN ML.

FIG. 4E

FIG.5A

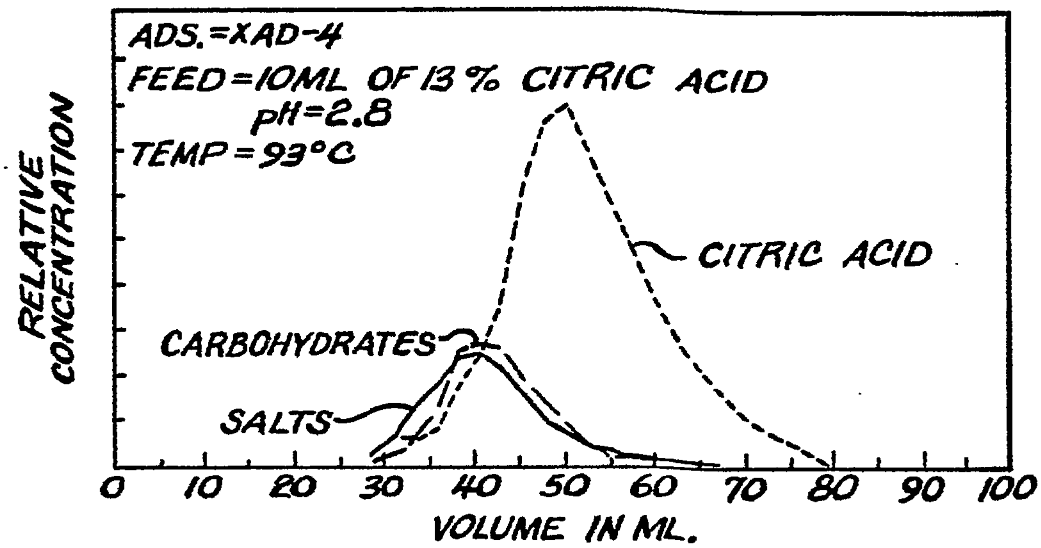
ADS.=XAD-4
FEED=10ML OF 13% CITRIC ACID
pH=2.8
TEMP=93°C
RELATIVE CONCENTRATION
CITRIC ACID
CARBOHYDRATES
SALTS
0 10 20 30 40 50 60 70 80 90 100
VOLUME IN ML.

FIG.5B

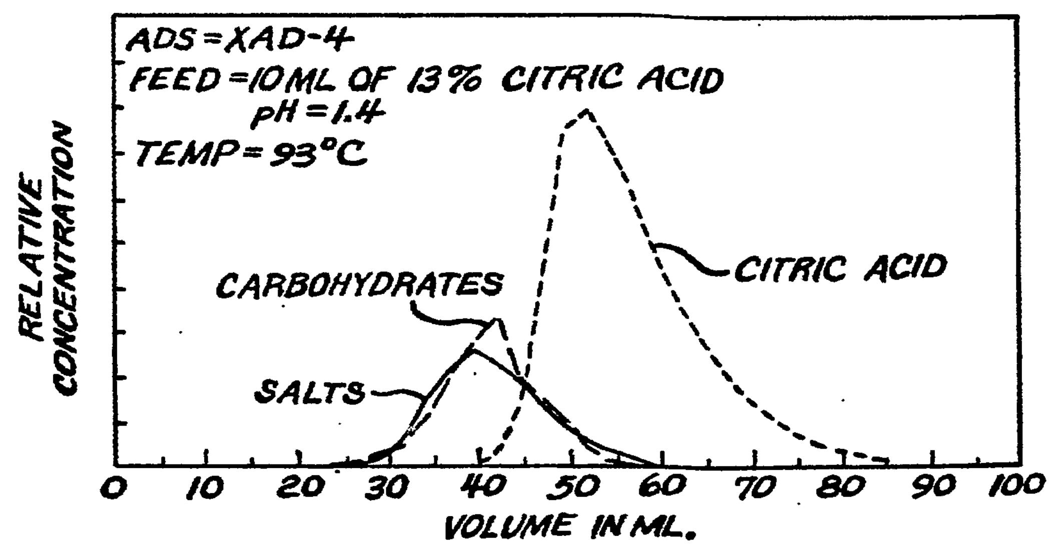
ADS=XAD-4
FEED=10ML OF 13% CITRIC ACID
pH=1.4
TEMP=93°C
RELATIVE CONCENTRATION
CITRIC ACID
CARBOHYDRATES
SALTS
0 10 20 30 40 50 60 70 80 90 100
VOLUME IN ML.

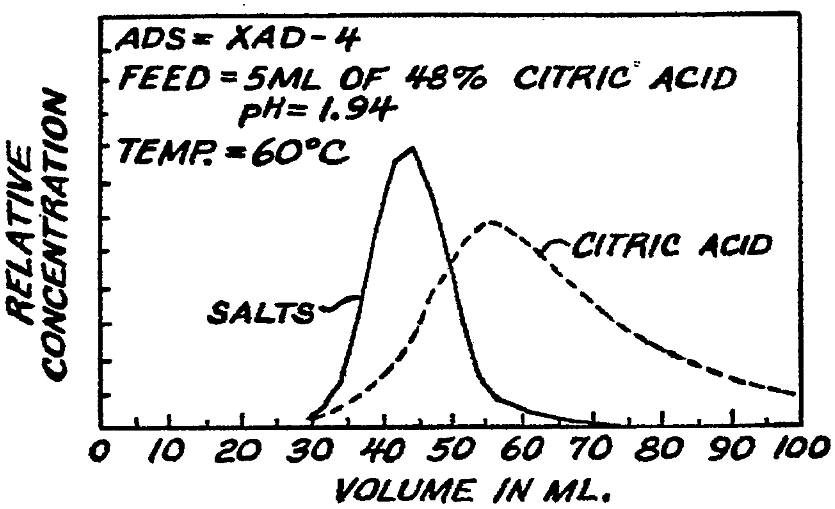


FIG. 6A

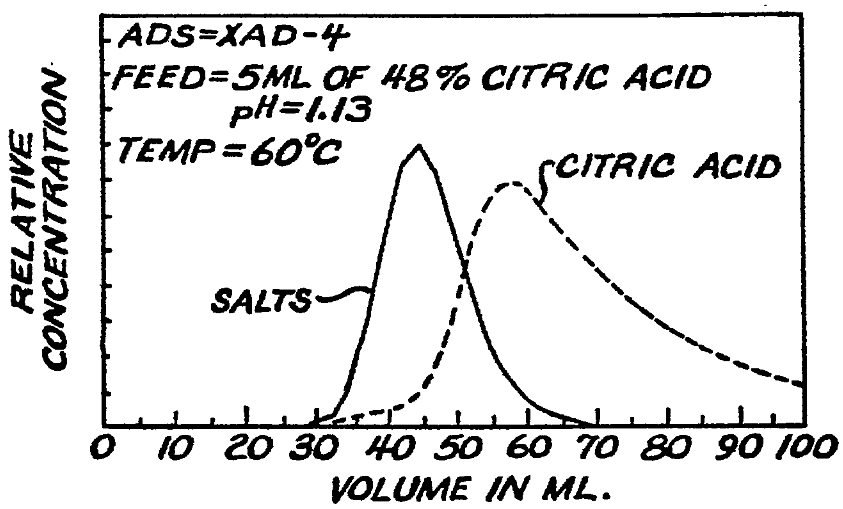


FIG. 6B

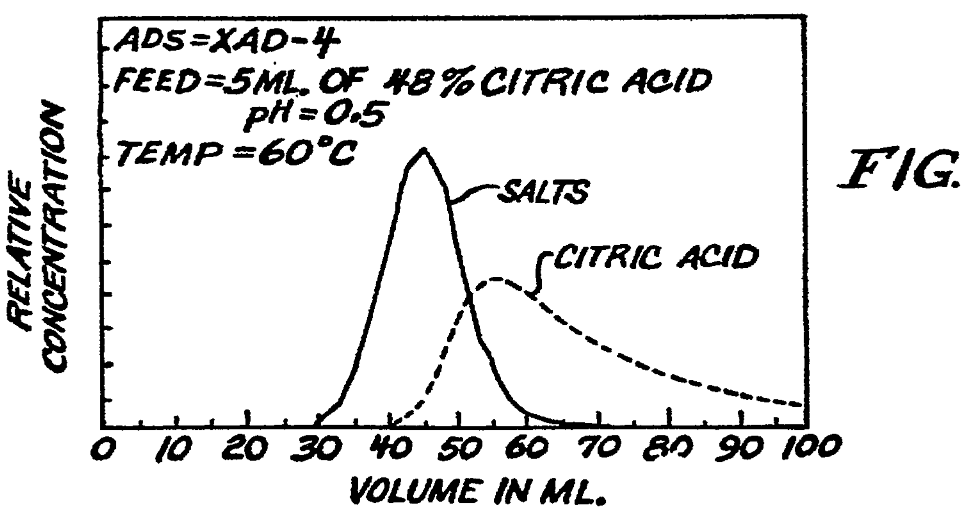


FIG. 6C

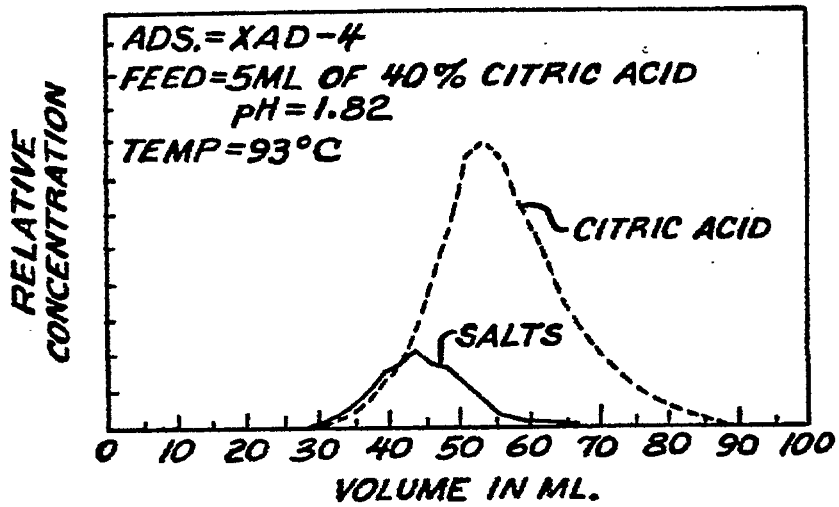


FIG.7A

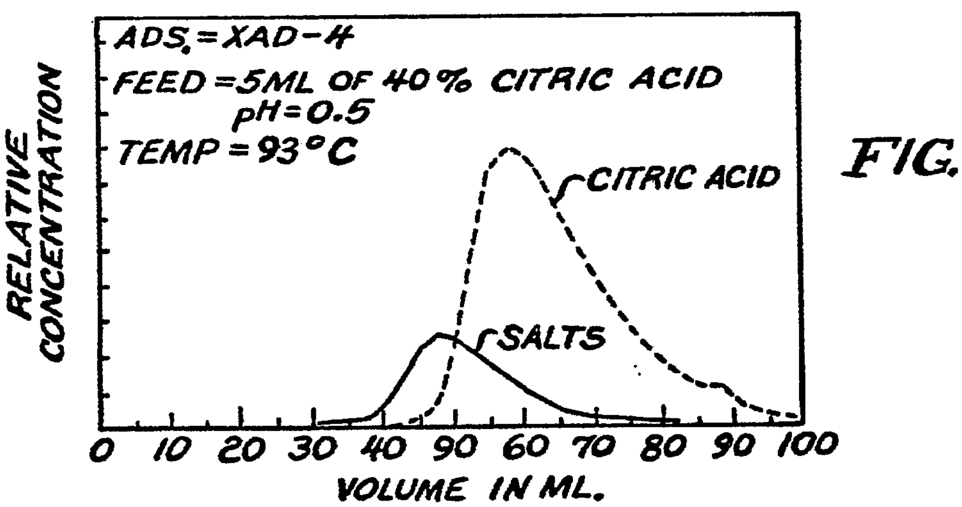


FIG.7B

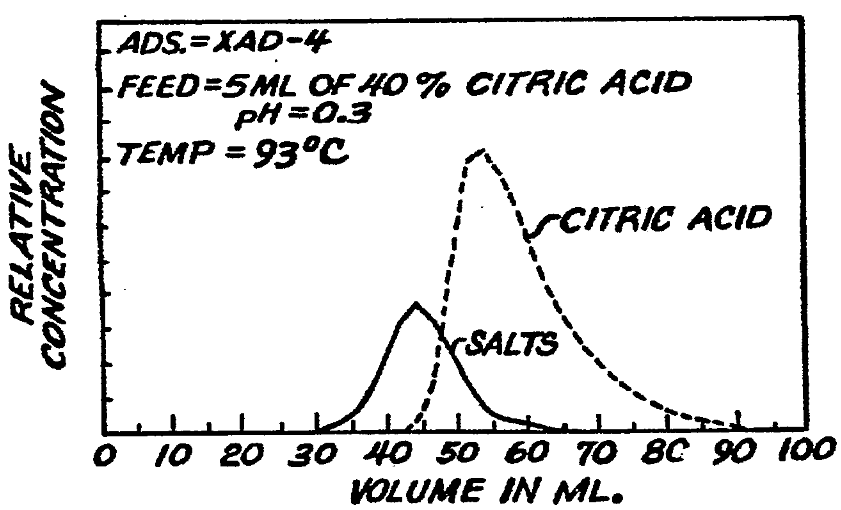


FIG.7C

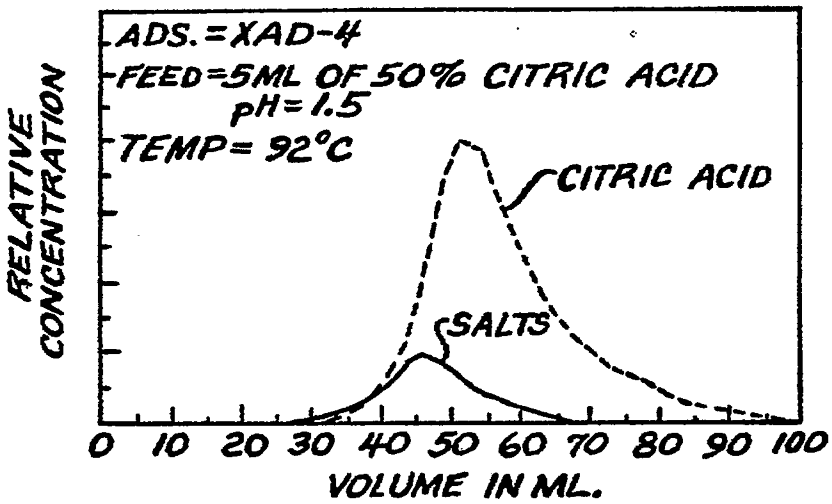


FIG.8A

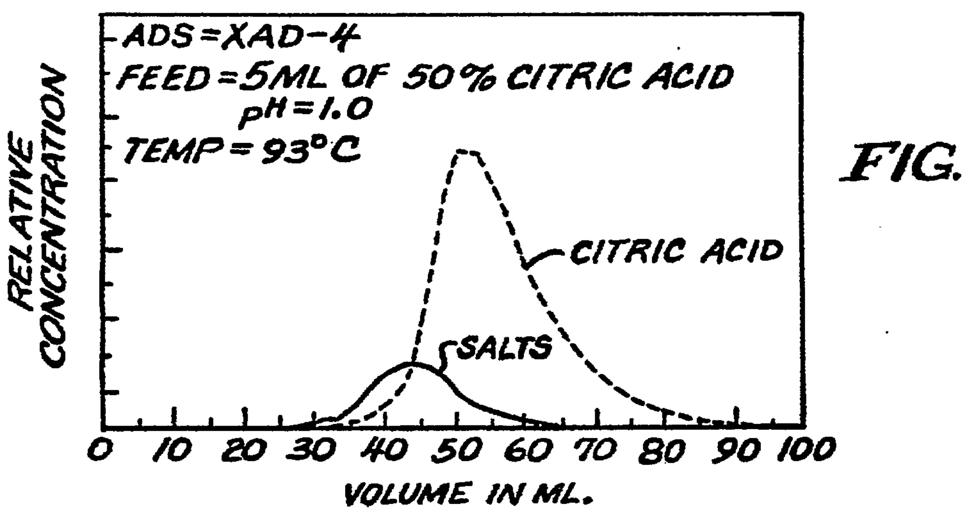


FIG.8B

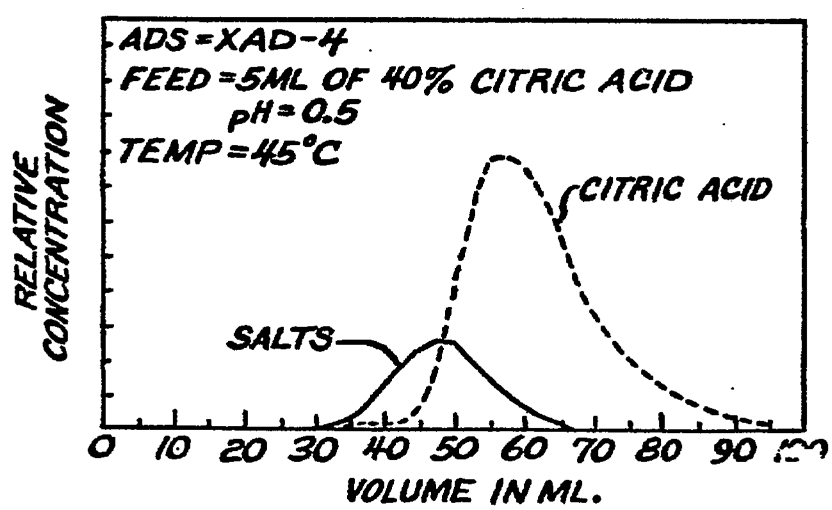


FIG.9

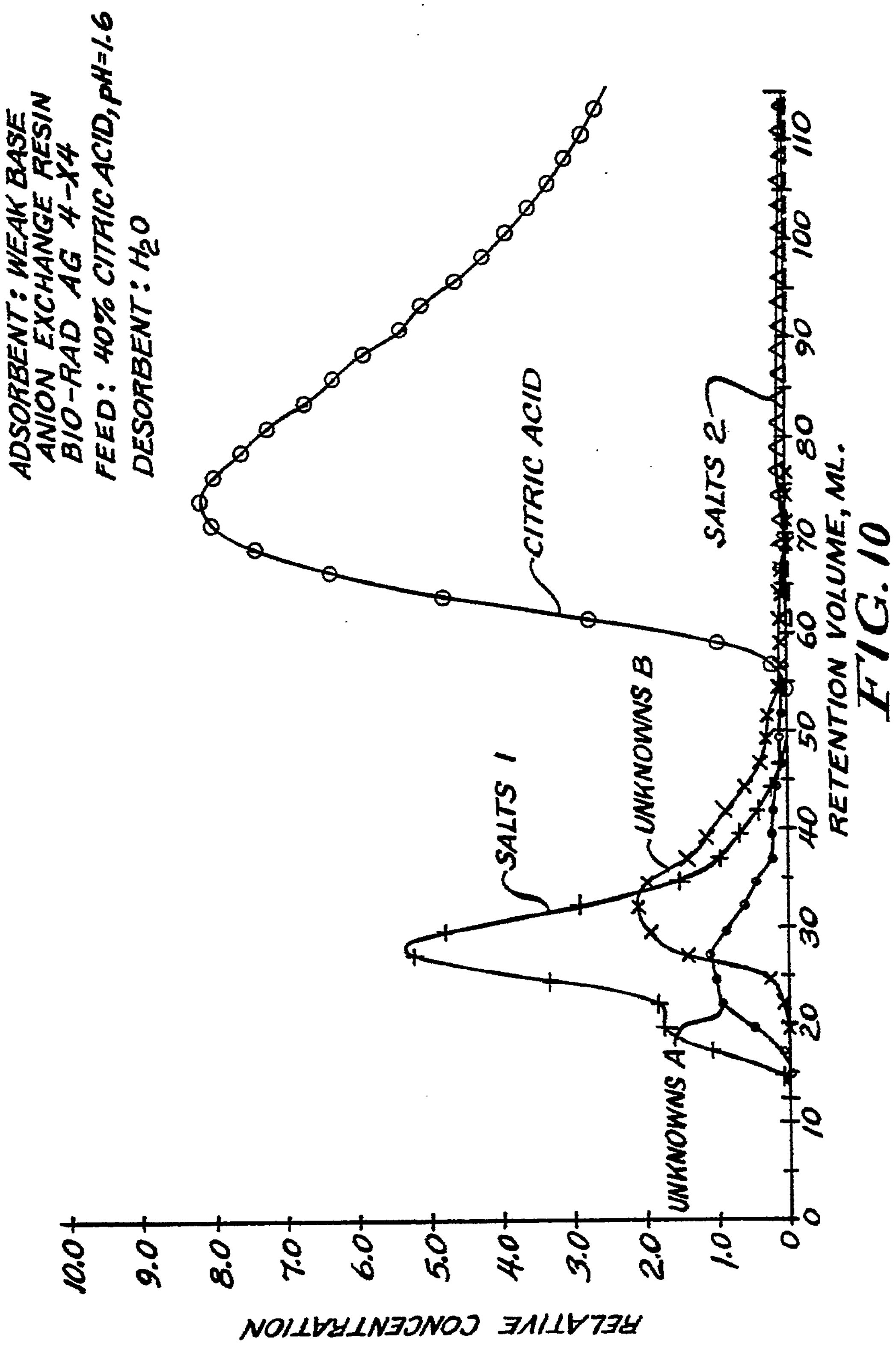
ADSORBENT: WEAK BASE ANION EXCHANGE RESIN BIO-RAD AG 4-X4
FEED: 40% CITRIC ACID, pH=1.6
DESORBENT: $H_2O$
CITRIC ACID
SALTS 1
UNKNOWNS B
UNKNOWNS A
SALTS 2
RELATIVE CONCENTRATION
RETENTION VOLUME, ML.
0
1.0
2.0
3.0
4.0
5.0
6.0
7.0
8.0
9.0
10.0
0
10
20
30
40
50
60
70
80
90
100
110

FIG. 10

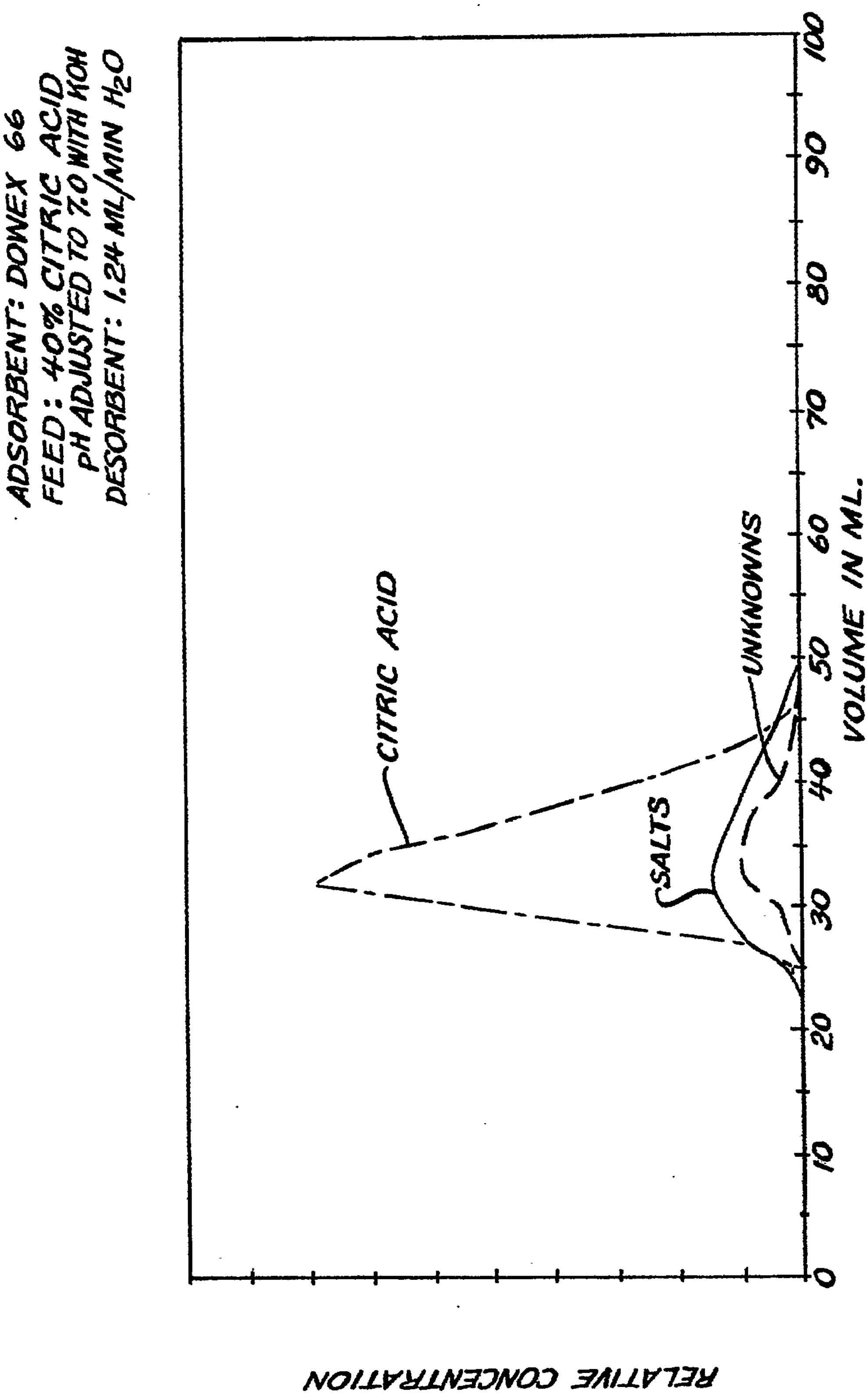
ADSORBENT: DOWEX 66
FEED: 40% CITRIC ACID
pH ADJUSTED TO 7.0 WITH KOH
DESORBENT: 1.24 ML/MIN H2O
CITRIC ACID
SALTS
UNKNOWNS
0
10
20
30
40
50
60
70
80
90
100
VOLUME IN ML.
RELATIVE CONCENTRATION

FIG. 11A

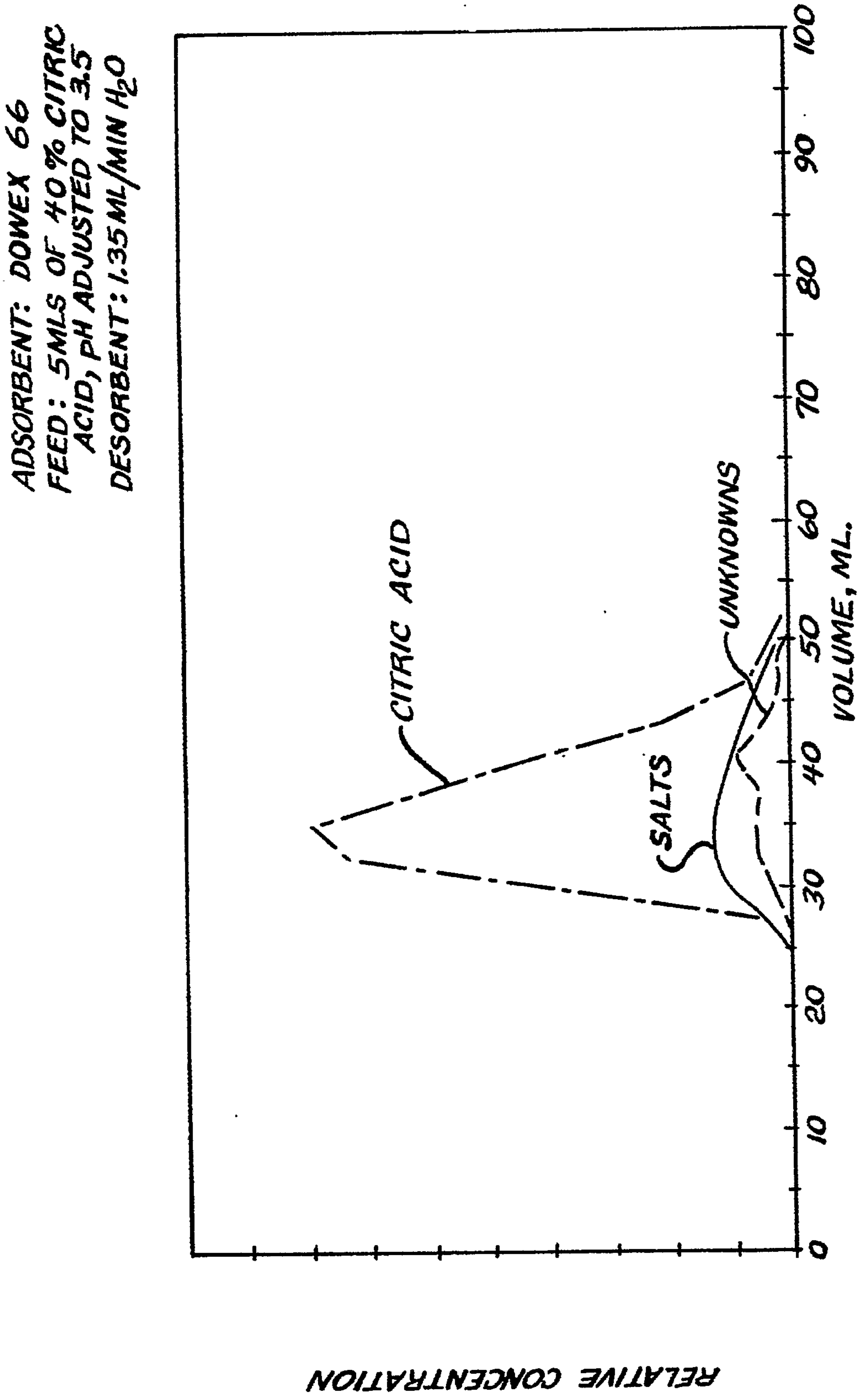
ADSORBENT: DOWEX 66
FEED: 5MLS OF 40% CITRIC ACID, pH ADJUSTED TO 3.5
DESORBENT: 1.35 ML/MIN $H_2O$
CITRIC ACID
UNKNOWNS
SALTS
0
10
20
30
40
50
60
70
80
90
100
VOLUME, ML.
RELATIVE CONCENTRATION

FIG. 11B

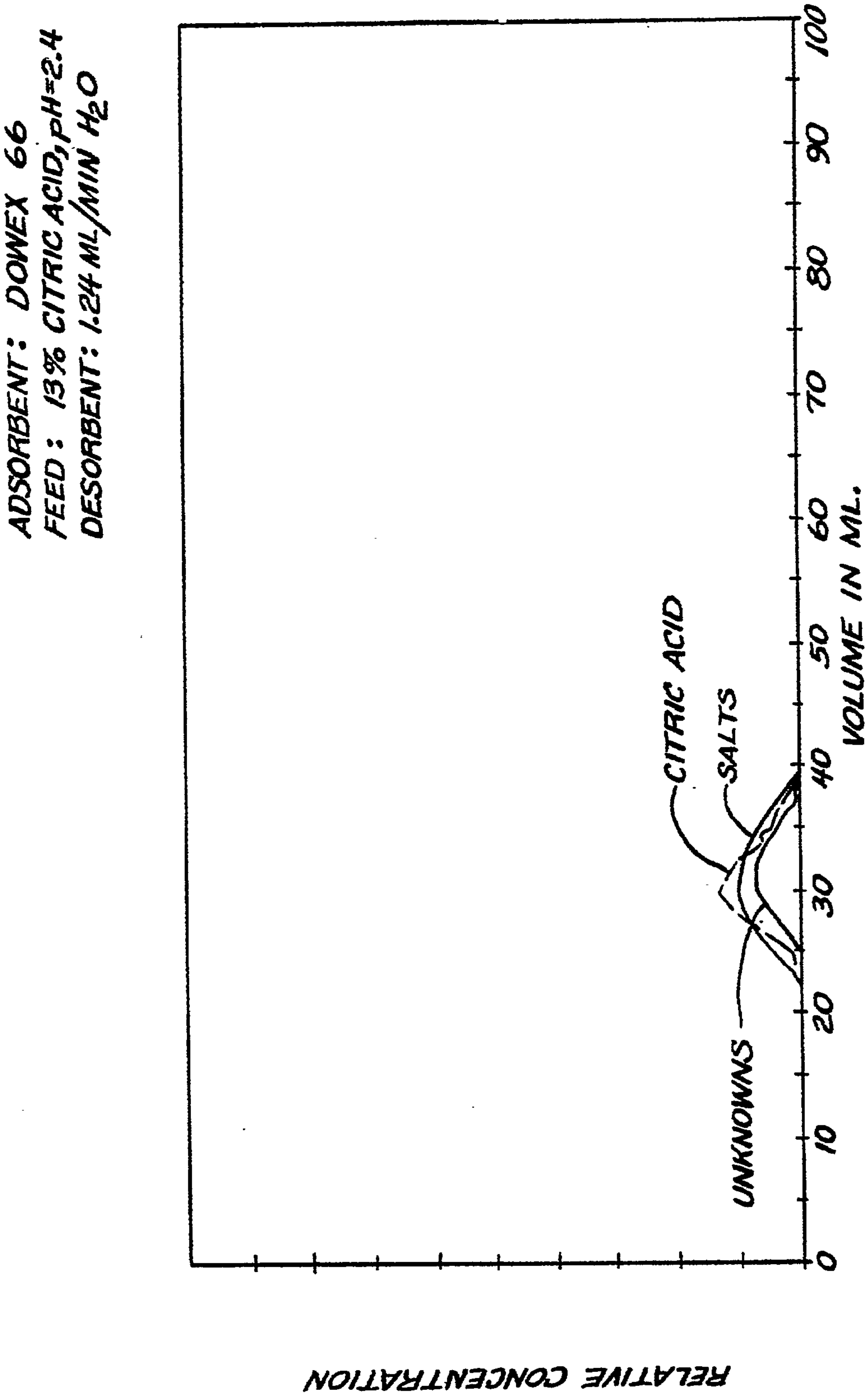
ADSORBENT: DOWEX 66
FEED: 13% CITRIC ACID, pH=2.4
DESORBENT: 1.24 ML/MIN $H_2O$
CITRIC ACID
SALTS
UNKNOWNS
0
10
20
30
40
50
60
70
80
90
100
VOLUME IN ML.
RELATIVE CONCENTRATION

FIG. 11C

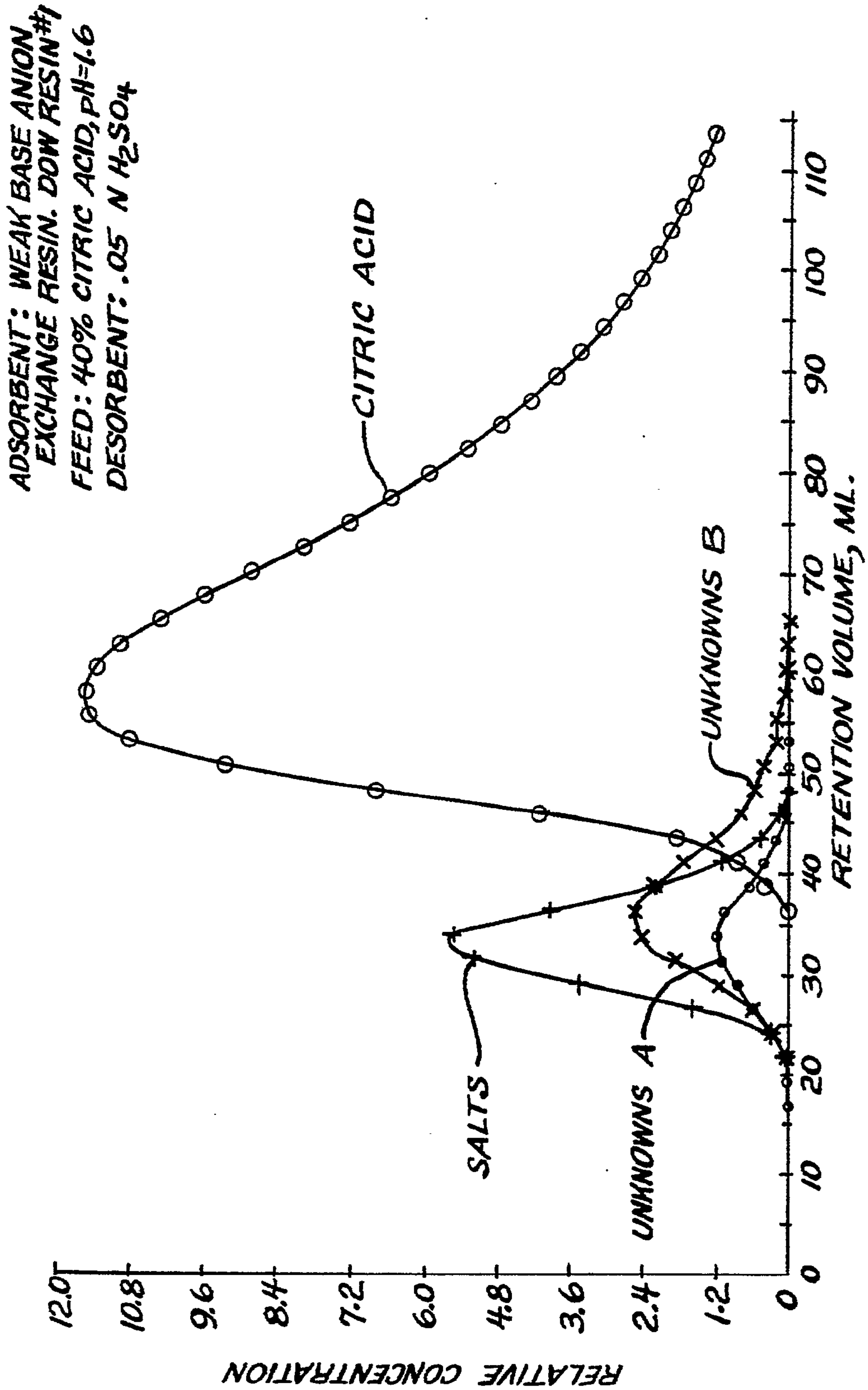
ADSORBENT: WEAK BASE ANION EXCHANGE RESIN. DOW RESIN #1
FEED: 40% CITRIC ACID, pH=1.6
DESORBENT: .05 N $H_2SO_4$
CITRIC ACID
SALTS
UNKNOWNS A
UNKNOWNS B
RELATIVE CONCENTRATION
RETENTION VOLUME, ML.
0
1.2
2.4
3.6
4.8
6.0
7.2
8.4
9.6
10.8
12.0
0
10
20
30
40
50
60
70
80
90
100
110

FIG. 12

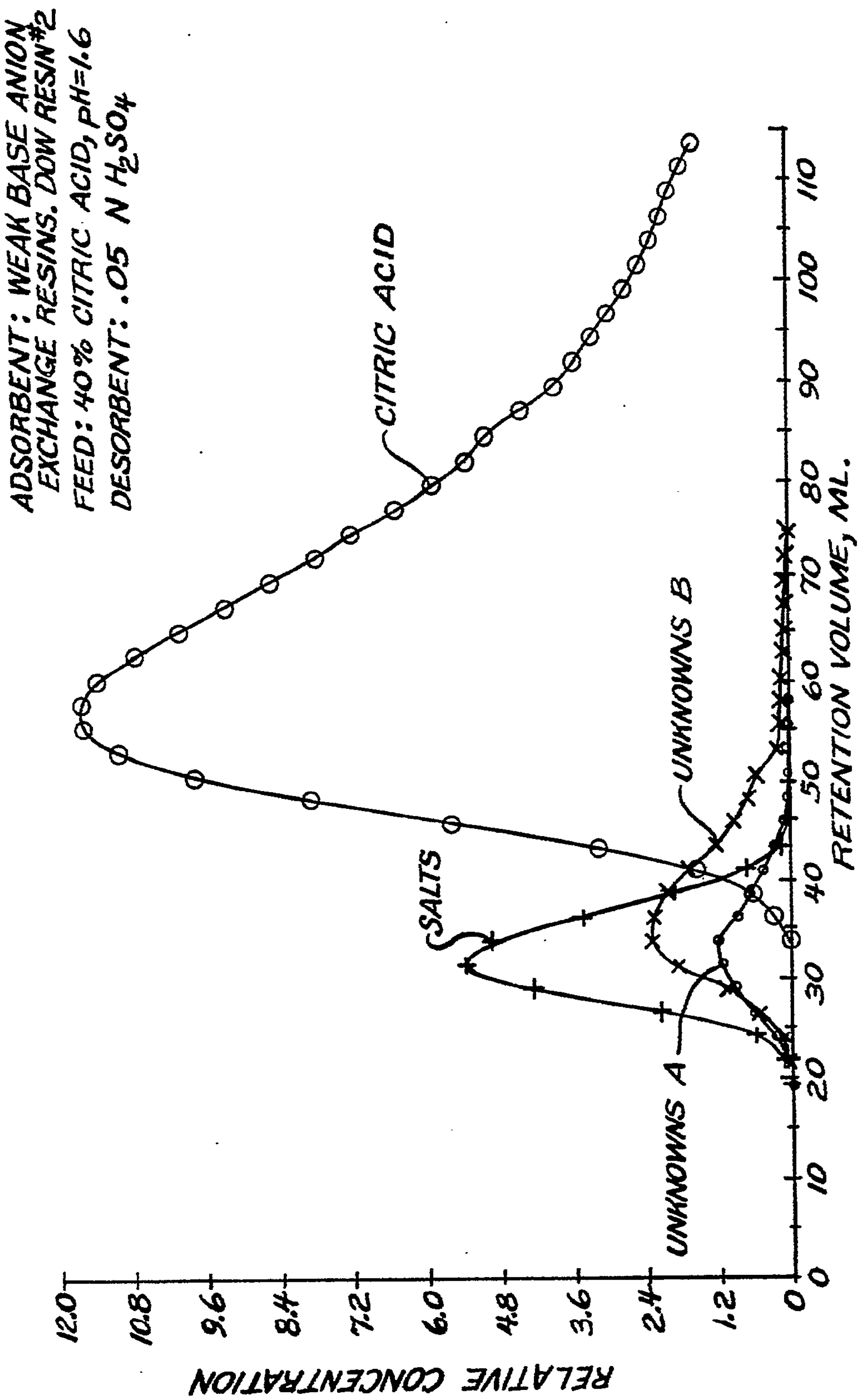
ADSORBENT: WEAK BASE ANION EXCHANGE RESINS. DOW RESIN #2
FEED: 40% CITRIC ACID, pH=1.6
DESORBENT: .05 N $H_2SO_4$
CITRIC ACID
SALTS
UNKNOWNS A
UNKNOWNS B
RELATIVE CONCENTRATION
0
1.2
2.4
3.6
4.8
6.0
7.2
8.4
9.6
10.8
12.0
RETENTION VOLUME, ML.
0
10
20
30
40
50
60
70
80
90
100
110

FIG. 13A

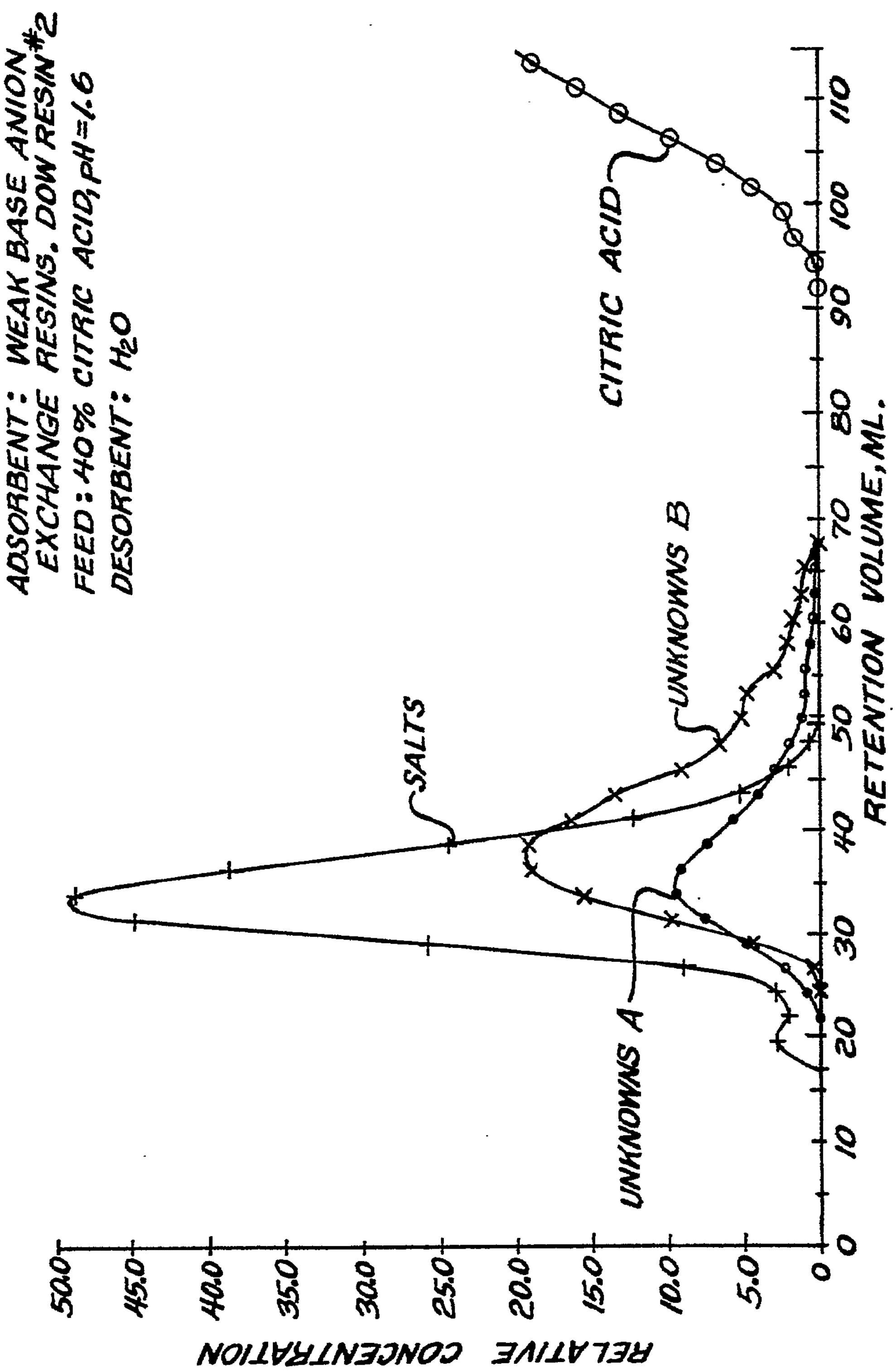
ADSORBENT: WEAK BASE ANION EXCHANGE RESINS. DOW RESIN #2
FEED: 40% CITRIC ACID, pH=1.6
DESORBENT: $H_2O$
SALTS
UNKNOWNS B
UNKNOWNS A
CITRIC ACID
RELATIVE CONCENTRATION
RETENTION VOLUME, ML.
50.0
45.0
40.0
35.0
30.0
25.0
20.0
15.0
10.0
5.0
0
10
20
30
40
50
60
70
80
90
100
110

FIG. 13B

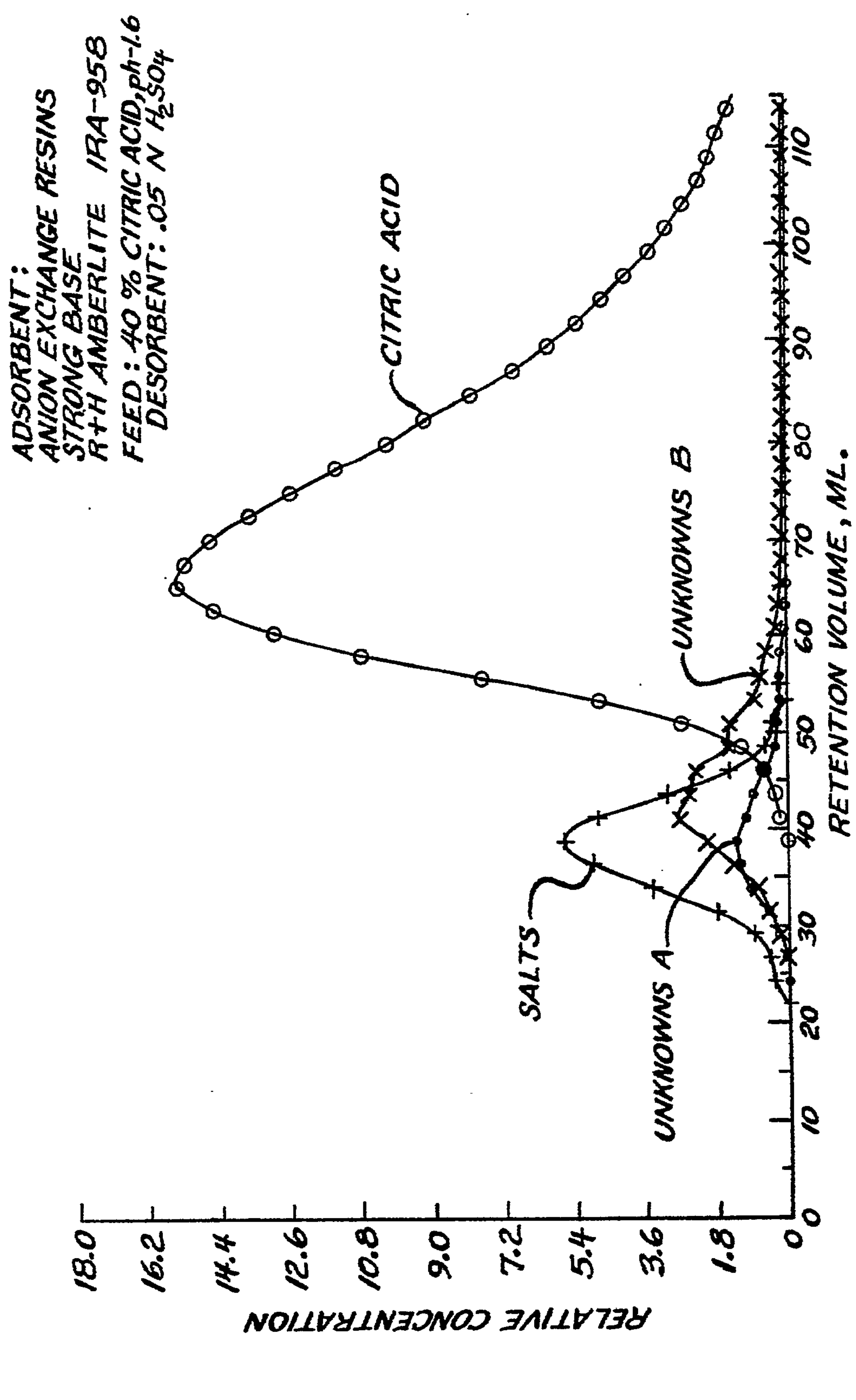
ADSORBENT:
ANION EXCHANGE RESINS
STRONG BASE
R+H AMBERLITE IRA-958
FEED: 40 % CITRIC ACID, ph-1.6
DESORBENT: .05 N $H_2SO_4$
CITRIC ACID
UNKNOWNS B
SALTS
UNKNOWNS A
RELATIVE CONCENTRATION
18.0
16.2
14.4
12.6
10.8
9.0
7.2
5.4
3.6
1.8
0
0
10
20
30
40
50
60
70
80
90
100
110
RETENTION VOLUME, ML.

FIG. 14

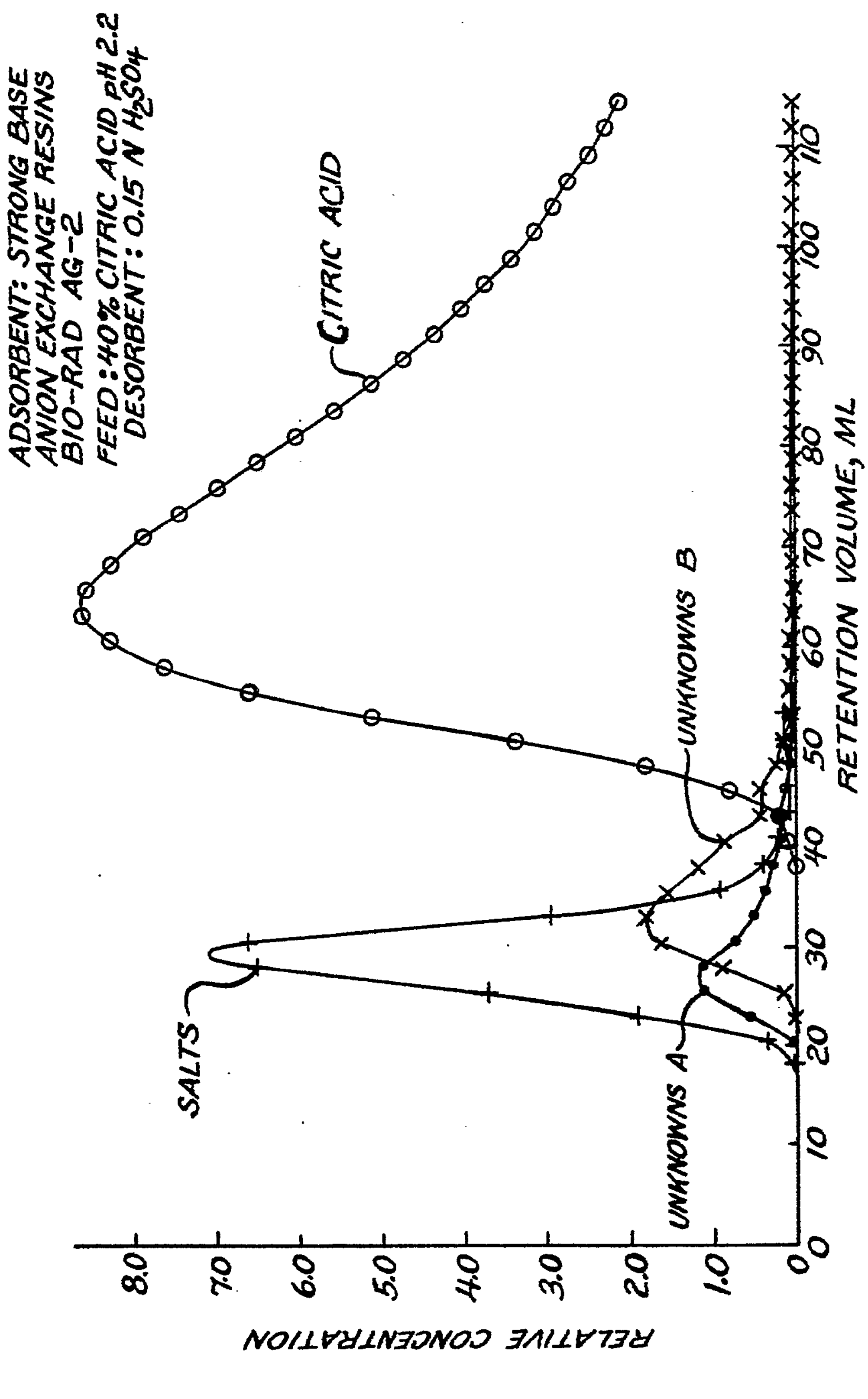
ADSORBENT: STRONG BASE
ANION EXCHANGE RESINS
BIO-RAD AG-2
FEED: 40% CITRIC ACID pH 2.2
DESORBENT: 0.15 N $H_2SO_4$
CITRIC ACID
SALTS
UNKNOWNS A
UNKNOWNS B
RELATIVE CONCENTRATION
0
1.0
2.0
3.0
4.0
5.0
6.0
7.0
8.0
0
10
20
30
40
50
60
70
80
90
100
110
RETENTION VOLUME, ML

FIG. 15